# EUROPEAN PATENT SPECIFICATION

(11) **EP 0 404 605 B1**
(45) Date of publication and mention of the grant of the patent: **13.11.1996**
(21) Application number: 90306942.5
(22) Date of filing: 25.06.1990
(51) Int. Cl.: C07F 9/30, C07F 9/32, A61K 51/10, A61K 39/395

(54) **Tri-aza macrocycles and processes for their production**
Tri-aza-Makrocyclen und Verfahren zu ihrer Herstellung
Tri-aza-macrocycles et leurs procédés de préparation

(30) Priority: 23.06.1989 GB 8914543
(43) Date of publication of application: 27.12.1990
(73) Proprietor: CELLTECH THERAPEUTICS LIMITED, Slough, Berkshire SL1 4EN (GB)
(72) Inventor: Parker, David, Durham, DH1 4DG (GB); Eaton, Michael Anthony William, Aston Rowant, Oxfordshire, OX9 5SH (GB)
(74) Representative: Hallybone, Huw George

(56) References cited:
- EP-A- 0 197 437
- WO-A-86/02352
- WO-A-89/01475
- BULLETIN OF THE ACADEMY OF SCIENCES OF THE USSR; CHEMICAL SCIENCES DIVISION, vol. 33, April 1984, pages 769-776; M.I. KABACHNIK et al.: "Synthesis and study of a new complexone - N,N',N''-tris-(dihydroxyphosphorylmethyl)-1,4,7- triazacyclononane"

## Description

This invention relates to functionalised tri-aza macrocycles, to metal complexes thereof, to conjugate compounds containing the functionalised tri-aza macrocycles and metal complexes thereof, and to their use in diagnosis and therapy.

The attachment of metal ions to proteins, peptides and other, smaller molecules is a fast expanding technology, which has numerous proven and potential applications in research, in industry and, particularly, in medicine.

In recent years, much of the impetus behind the development of this technology has been the ability to link metal ions to antibodies, especially monoclonal antibodies. Such metal labelled antibodies have found a widespread use, especially in medicine, where they have been employed, for example, to target the metal ions to a specific tissue type, both in vitro and in vivo. Thus, metal labelled antibodies have applications in locating specific tissue types (e.g. employing computer-aided tomographic techniques where the metal ion is in some way detectable) and in the treatment of cell disorders (e.g. treating mammalian tumours where the metal ion is a cytotoxic radionuclide).

Conventionally, attachment of the metal ion to a protein such as an antibody has been achieved by complexation by an acyclic chelate such as a substituted diethylenetriaminepentaacetic acid [Gansow O. A. et al, Inorg. Chem., (1986), 25, 2772, and see for example, U.S. patent No. 4454106] or ethylenediaminetetraacetic acid [Meares, C. F. et al, Acc. Chem. Res., (1984), 17, 202] covalently linked to the antibody. Such acyclic complexes however tend to be unstable in vivo either as a result of acid-catalysed decomplexation or competitive chelate binding by Ca²⁺ or Zn²⁺ in serum, or as a result of competition from transferrin [Moerlein, S. M. et al, Int. J. Nuc. Med. Biol., (1981) 8, 277]. The lack of stability can result in uncomplexed metal atoms in the body which have a cytotoxic effect on healthy tissue (e.g. bone marrow) or which markedly reduce the signal-to-noise ratio of an imaging technique.

A possible alternative to the use of acyclic chelates in the labelling of antibodies is the use of macrocyclic ligands, which has previously been suggested in broad terms [Gansow O. A. et al, Am. Chem. Soc. Symp. Ser., (1984), 241, 215; UK Patent Specification No. 2122641; and Moi M. K. et al, Anal. Biochem., (1985), 148, 249-253]. More recently, tri-aza and tetra-aza macrocycles have been described which are capable of binding metals, and which can be conjugated to antibodies (International Patent Specifications Nos. WO-A-8901475 and WO-A-8901476).

Other tri-aza macrocycles have also been described, which are capable of binding metals [International Patent Specification No. WO-A-8602352; European Patent Specification No. 197437; Bryden, C.C. et al, Rare Earths Mod. Sci. Technol. (1982), 3, 53-57; Kabachnik, I.M. et al, Izv. Akad. Nauk. SSSR, Ser. Khim., 835 (1984)]. Some compounds of these types, when complexed with metals, have been proposed for use as contrast agents for use in diagnostic imaging.

We have now found a new class of functionalised tri-aza macrocycles, members of which are able to form kinetically inert complexes with metal ions which are of use in diagnosis and therapy.

### Summary of the Invention

Thus according to one aspect of the present invention we provide a compound of general formula (1): 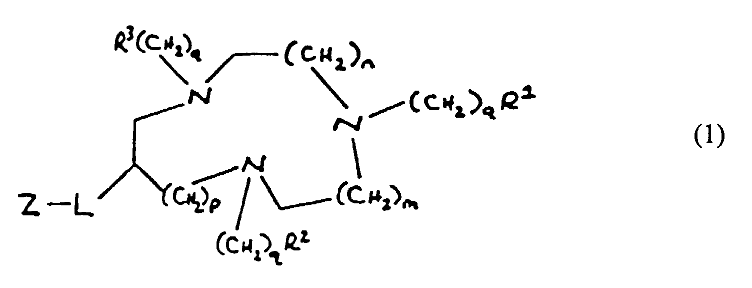 wherein
m and n, which may be the same or different, is each zero or an integer 1, 2, or 3;
p is zero or an integer 1 or 2;
q is zero or an integer from 1 to 6 inclusive;
R¹, R² and R³, which may be the same or different, is each a hydrogen atom or a group -CO₂H or -P(O)(XH)R⁴ (where X is an oxygen or sulphur atom and R⁴ is a hydrogen atom or an alkyl or alkoxy group), with the proviso that at least one of R¹, R² and R³ is a -P(O)(XH)R⁴ group;
L is a covalent bond or a linker group;
Z is a hydrogen atom or a reactive functional group capable of reacting with a thiol, amino, carboxyl, hydroxyl, aldehyde, aromatic or heteroaromatic group;
and metal complexes and/or salts thereof.

In the compounds of formula (1), alkyl groups represented by R⁴ may be for example C₁₋₆alkyl groups such as methyl or ethyl groups.

Alkoxy groups represented by R⁴ may be C₁₋₆alkoxy groups such as methoxy or ethoxy groups.

In general, compounds of formula (1) in which R¹, R² and R³ are the same and is each a group -P(O)(XH)R⁴ are preferred. Compounds of this type in which q is an integer from 1 to 6 inclusive, particularly an integer 1, are especially preferred. Particularly useful compounds of formula (1) are those wherein R¹, R² and R³ is each a group -P(O)(OH)H, -P(O)(OH)OCH₃, -P(O)(OH)OCH₂CH₃ or especially -P(O)(OH)R⁴ where R⁴ an alkyl group, particularly a methyl group. In compounds of this type, q is preferably an integer from 1 to 6 inclusive, particularly an integer 1.

In the compounds of formula (1), it will be appreciated that the nature of the group L when it is a linker group may be varied widely without substantially affecting the usefulness of compounds of formula (1) and the metal complexes thereof. Thus L may be any suitable organic radical and may be for example an optionally substituted aliphatic hydrocarbyl chain, optionally interrupted by one or more heteroatoms selected from -O- or -S- or by one or more -N(R⁵)- (where R⁵ is a hydrogen atom or a C₁₋₆alkyl group), -CON(R⁵)-, -N(R⁵)CO-, cycloaliphatic, aromatic, or heteroaromatic groups.

In the above definition, and in the same context whenever it appears below, the term "interrupted by" as applied to cycloaliphatic or aromatic groups is to be understood to also mean that these particular groups may additionally be present linked to the terminal carbon atom of the hydrocarbyl chain represented by L, at the opposite end of the chain to the carbon atom attached to the macrocycle.

Thus, for example, L may be an optionally substituted straight or branched C₁₋₂₀alkylene, C₂₋₂₀alkenylene, or C₂₋₂₀alkynylene chain, optionally interrupted by one or more -O- or -S- atoms or C₅₋₈cycloalkylene (e.g. cylcopentylene or cyclohexylene), C₆₋₁₂aromatic (e.g. phenylene or substituted phenylene), C₅₋₁₀heteroaromatic (e.g. furanyl, pyridyl), -N(R⁵)-, -CON(R⁵)- or -N(R)⁵CO- groups.

Examples of substituents which may be present on the chain L include halogen atoms, e.g. fluorine, chlorine, bromine, or iodine atoms or groups selected from C₁₋₆alkoxy (e.g. methoxy or ethoxy), hydroxy, nitro, -N(R⁶)(R⁷), [where R⁶ is a hydrogen atom or a C₁₋₆alkyl group and R⁷ is a C₁₋₆alkyl group; e.g. -NHCH₃ or -N(CH₃)₂], or substituted amido, e.g. a group of formula -(CH₂)ₙCON(R⁸)(R⁹) [where n is zero or an integer 1 to 4 inclusive, R⁸ is a hydrogen atom or a C₁₋₆alkyl group, e.g. methyl and R⁹ is an optionally substituted C₁₋₆alkyl group].

Substituted alkyl groups represented by R⁹ include for example C₁₋₆alkyl groups substituted by one or more halogen atoms, or nitro, amino or hydroxy groups.

In general, in compounds of formula (1) the linker group is preferably an optionally substituted C₁₋₁₀alkylene, (especially C₁₋₆alkylene such as methylene, ethylene, propylene butylene, pentylene or hexylene) C₂₋₁₀alkenylene or C₂₋₁₀alkynylene chain optionally interrupted by one or more -O- or -S- atoms or cyclohexylene, phenylene, substituted phenylene, -NH-, -N(CH₃)-, -CONH-, -CONH(CH₃)- -NHCO- or -N(CH₃)CO- groups.

Particular examples of linker groups represented by L include, for example, -(CH₂)_{d}- (where d is an integer 1 to 4 inclusive), 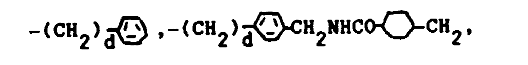 -(CH₂)_{d}NHCO(CH₂)ₑ- (where e is an integer 1 to 4 inclusive) and -(CH₂)_{d}NHCO(CH₂)ₑOCH₂-.

The reactive functional group represented by Z in compounds of formula (1) may be any group capable of reacting with a thiol, amino, carboxyl, hydroxyl aldehyde, aromatic or heteroaromatic group. Aromatic groups include, for example, phenolic groups. Heteroaromatic groups include for example imidazolyl groups.

Thus, Z may be, for example, a halogen atom, for example a chlorine, bromine or iodine atom or a group selected from -SH, -NH₂, hydrazine (-NHNH₂) or a derivative thereof, [for example -N(CH₃)NH₂, -NHCONHNH₂, -NHCSNHNH₂, or phenyl hydrazine], -NCO, -NCS, -COR¹⁰, [where R¹⁰ is a halogen atom such as a chlorine or bromine atom, or a N₃, C₁₋₆alkoxy, e.g. methoxy, C₆₋₁₂aryloxy (e.g. nitrophenyloxy or dinitrophenyloxy), imidyloxy (e.g. succinimidyloxy) or imidazolyoxy group], imide, e.g. maleimide, a vinyl group of formula -Het¹-C(Het²)=CH₂ (where Het¹ and Het², which may be the same or different, is each a nitrogen containing heterocyclic group, e.g a pyridyl group or Het¹ is a nitrogen containing heterocyclic group and Het² is a hydrogen atom) for example a vinyl pyridyl group of formula 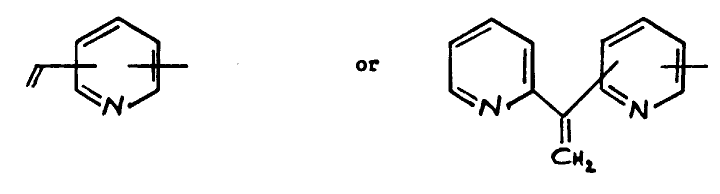 especially, 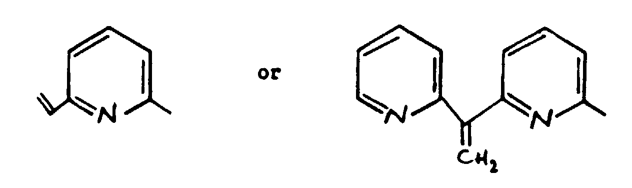 or a dione of formula 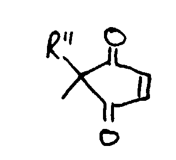 (where R¹¹ is a C₁₋₄ alkyl e.g. methyl, group).

Metal complexes of the compounds of formula (1) include complexes wherein the metal is di- or tripositive and has a coordination number from 2 up to 6, especially 6. The metal may be a radioactive isotope. Examples of suitable metals include indium (In), copper(Cu), lead (Pb), bismuth (Bi), cobalt (Co) and gallium (Ga). In, Ga, Co, and Cu are preferred, particularly In and Ga. ¹¹¹In, ⁶⁹Ga and ⁷¹Ga are particularly preferred.

In general, optimum binding of the metal to the compounds of formula (1) may be achieved by selection of the ring size and where appropriate by adjusting the potential coordination number by choice of the group -(CH₂)_{q}R¹, -(CH₂)_{q}R², and/or -(CH₂)_{q}R³. Thus a particularly important class of compound of formula (1) is that wherein p is zero. Especially useful compounds are those wherein p is zero, m is an integer 1 and n is an integer 1. In general, compounds of formula (1) in which -(CH₂)_{q}R¹, -(CH₂)R² and -(CH₂)_{q}R³ is each -CH₂P(O)(OH)R⁴ - where R⁴ is -H, -OCH₃, -OCH₂CH₃ or an alkyl group, especially a methyl group, are particularly useful.

Salts of the compounds of formula (1) include salts with inorganic or organic bases, for example alkali metal or alkaline earth metal salts such as lithium, sodium, potassium, magnesium or calcium salts; amine salts, such as those from primary, secondary or tertiary amines, for example ethanolamine, diethanolamine, morpholine, glucamine, N-methylglucamine or N-N-dimethylglucamine salts; and amino acid salts such as lysine, arginine and ornithine salts; or acid addition salts such as hydrobromides or hydrochlorides. Pharmaceutically acceptable salts are particularly preferred.

An important group of compounds according to the invention has the formulae (1a): 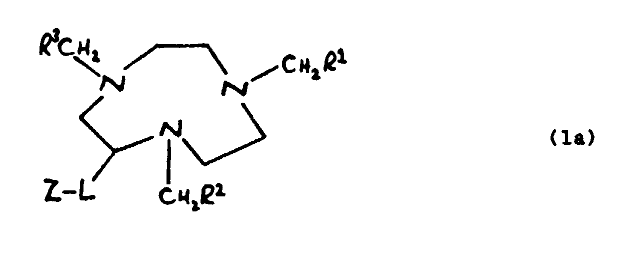 wherein R¹, R², R³, L and Z are as defined for formula (1) and metal complexes and/or salts thereof.

Compounds of this type in which R¹, R², and R³ is each P(O)(OH)R⁴ where R⁴ is -H, -OCH₂CH₃ or, especially -CH₃ are particularly preferred.

Compounds of formula (1a) in which L is a linker group [particularly those specifically identified for compounds of formula (1)] are especially useful. Particular groups of this type are those of formulae -(CH₂)_{d}- and -(CH₂)_{d}NHCO(CH₂)ₑ-, where d and e, which may be the same or different is each an integer 1 to 4 inclusive.

Z in compounds of formula (1a) is preferably a reactive functional group, [particularly those specifically identified for compounds of formula (1)], especially a group of formula -NH₂, -COR¹⁰ -Het¹-C(Het²)=CH₂ or a dione of formula: 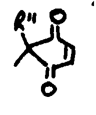 Indium and gallium complexes of the compounds of formula (1a) are particularly useful.

A further group of compounds of formula (1a) which is particularly useful is that wherein L in the compounds is a covalent bond, and Z is a hydrogen atom, and metal complexes and/or salts thereof. Gallium complexes of compounds of this type are particularly important, especially ⁷¹Ga complexes, and particularly gallium complexes where R¹, R², and R³ is each P(O)(OH)R⁴, especially P(O)(OH)CH₃.

The compounds of formula (1) and the metal complexes and/or salts thereof have a diagnostic use, for example as imaging agents in vitro and in vivo. The compounds of formula (1a) and the salts thereof are especially useful for use as imaging agents, particularly the gallium complexes, which are particularly useful in nuclear magnetic reasonance imaging, especially the gallium (particularly ⁷¹Ga) complexes of formula (1a) wherein L is a covalent bond, Z is a hydrogen atom and R¹, R² and R³ is each a group P(O)(OH)R⁴ especially P(O)(OH)CH₃ and the salts thereof

The compounds of formula (1) and the metal complexes and/or salts thereof are also cytotoxic agents and may be used in the treatment of abnormal cell disorders, for example in the treatment of tumours. For use as diagnostic and/or therapeutic agents, the compounds may be employed using conventional methods (e.g. for formulation and presentation), already is use for metal complexing reagents.

For application of the compounds of formula (1) as imaging or cytotoxic agents, it may be preferable to couple the compounds to other molecules such as proteins, especially antibodies, peptides or carbohydrates to form conjugate compounds, and the compounds of formula (1) are particularly well adapted for use in this respect.

The compound of formula (1) may be coupled through any thiol, amino, carboxyl, hydroxyl, aldehyde, aromatic or heteroaromatic group present in the protein, peptide or carbohydrate.

Thus in a further aspect of the invention, we provide a conjugate compound which comprises a compound of formula (1) or a metal complex and/or salt thereof, coupled to a protein, peptide or carbohydrate.

It is to be understood that a conjugate compound according to the invention may comprise more than one molecule of a compound of formula (1) coupled to any one protein, peptide or carbohydrate molecule.

In a particular aspect, the invention provides a conjugate compound of formula (2): 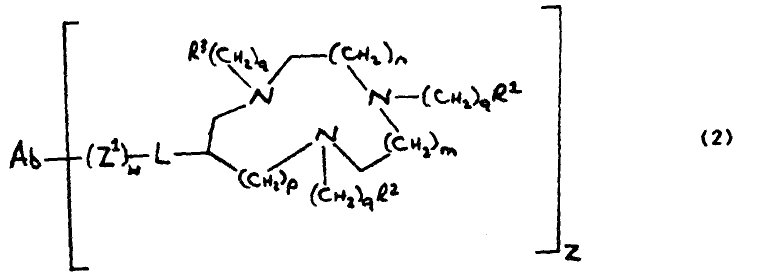 wherein m, n, p, q, R¹, R², R³, and L are as defined for formula (1);
Z¹ is the residue of a reactive functional group;
w is zero or an integer 1;
z is an integer 1 or more;
Ab is an antibody; and metal complexes and/or salts thereof.

In the compound of formula (2), the residue or a reactive functional group represented by Z¹ may in general be the residue of a reactive functional group Z as defined for formula (1).

In particular, Z¹ may be for example -S-, -NH- -NHN=, -N(CH₃)N=, -NHCONHN=, -NHCSNHN=, -N(Ph)N= (where Ph is phenyl), -NC(O)-, -NC(S), -CO-, 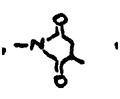 -Het¹ -C(Het²)CH₂- or

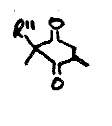

The antibody in the conjugates of formula (2) may in general belong to any immunoglobulin class. Thus for example it may be an immunoglobulin M antibody or, in partiuclar, an immunoglobulin G antibody. The antibody molecule may be of animal, for example mammalian origin, and may be for example of murine, rat or human origin. It may be a natural antibody or a fragment thereof, or, if desired, a recombinant antibody or antibody fragment i.e. an antibody molecule or antibody fragment which has been produced using recombinant DNA techniques.

Particular recombinant antibodies or antibody fragments include, (1) those having an antigen binding site at least part of which is derived from a different antibody, for example those in which the hypervariable or complementarity determining regions of one antibody have been grafted into the variable framework regions of a second, different antibody (as described in European Patent Specification EP-A-239400); (2) recombinant antibodies or fragments wherein non-Fv sequences have been substituted by non-Fv sequences from other, different antibodies (as described in European Patent Specification EP-A-171496, 173494 and 194276; or (3) recombinant antibodies or fragments possessing substantially the structure of a natural immunoglobulin but wherein the hinge region has a different number of cysteine residues from that found in the natural immunoglobulin, or wherein one or more cysteine residues in a surface pocket of the recombinant antibody or fragment is in the place of another amino acid residue present in the natural immunoglobulin (as described in International Patent Applications WO-A-89 01974 and WO-A-89 01782 respectively).

The antibody may be of polyclonal or, preferably, monoclonal origin. It may be specific for any number of antigenic determinants, but is preferably specific for one. The antigenic determinants may be any hapten or antigenic determinant associated with any antigen. Particular antigens include those associated with animals, e.g. humans, [for example normal animal tissue or organ cell-associated antigens, tumour cell-associated antigens (for example oncofetal antigens such as carcinoembryonic antigen or alphafetoprotein, placental antigens such as chorionic gonadotropin and placental alkaline phsophatase, and prostate antigens such as prostatic acid phsophatase and prostate specific antigen) and antigens associated with components of body fluids such as fibrin or platelets], viruses, bacteria and fungi.

In a preferred aspect the antibody may be capable of recognising and binding a tumour cell-associated antigen, particularly one or more epitopes on the TAG-72 antigen associated with human breast and colon tumours. A particularly preferred antibody of this type is the monoclonal antibody B72.3 [Colcher, D. et al Proc. Nat. Acad. Sci. USA (1981), 78 3199] or a fragment thereof, particularly a F(ab')₂ fragment.

The antibody Ab will in general be coupled to the remainder of the conjugate of formula (2) (i.e. the macrocycle and linker) through any appropriate reactive atom or group, for example a nitrogen or, especially, sulphur atom, present in the antibody. It will be appreciated that any one antibody molecule may contain more than one reactive group capable of coupling with the macrocycle and linker. Thus, for example, z in the conjugates of formula (2) may be an integer 1, 2, 3, 4, 5, 6 or more depending on the number of macrocycles linked to any particular antibody molecule or fragment.

Indium and gallium complexes of conjugates of formula (2) are particularly useful.

It is to be understood that the definitions and preferences expressed for m, n, p, q, R¹, R², R³ and L in compounds of formula (1), and for classes of compounds of formula (1) are also applicable to conjugates of formula (2).

Particularly useful conjugate compounds according to the invention are those comprising a compound of formula (1a), or a metal complex and/or salt thereof, coupled to an antibody. The indium and gallium complexes of these conjugates are especially important.

The compounds of formulae (1) and (2) may be formulated for use in accordance with conventional practice, and thus according to a further respect of the invention we provide a composition comprising a compound of formula (1) or a compound of formula (2) or a metal complex and/or salt thereof, together with one or more pharmaceutically acceptable carriers.

Particularly suitable compositions according to the invention are those adapted for parenteral administration, especially intravenous administration. Suitable formulations of this type include solutions of the compounds of formulae (1) or (2) in isotonic saline.

The quantities of compounds of formulae (1) or (2) used in formulations according to the invention will vary according to the intended use (i.e. imaging or therapy) and other variables such as the intended cell target, but may be easily determined in accordance with conventional practice for reagents of this type.

Compounds of the invention may be prepared by the following processes wherein the groups and symbols R¹, R², R³, m, n, p, q, L, Z, Ab and z are as defined for formulae (1) and (2) except where stated otherwise. Where a metal complex is desired as a final product, the complexation with a metal atom may be carried out as a final step in the production process, as described below for the complexation of compounds of formula (1), or alternatively it may be desirable to complex the metal at an earlier stage in the process, providing of course that the requisite macrocycle structure is present. In the following processes, it may be desirable to use starting materials in which the group 1 is in a protected state, or which contain a precursor of the group, as discussed below.

Thus, according to a further aspect of the invention a compound of formula (1) [wherein q is an integer 1-6 and, where present, in R¹, R² and/or R³ the group is an oxygen atom] or a metal complex thereof may be prepared by reaction of a corresponding compound of formula (3) 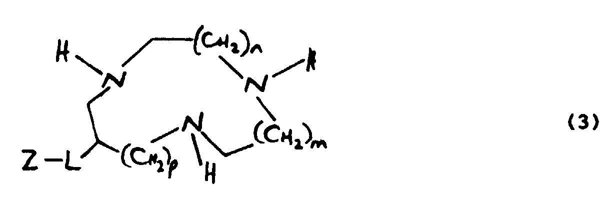 or a metal complex thereof, with a reagent
D(CH₂)_{q}P(O)(OR¹³)R⁴, or D(CH₂)_{q}P(O)(OR¹³)₂, (where D is a displaceable group, for example a halogen atom such as a bromine atom; R¹³ is a C₁₋₄alkyl, e.g. methyl or ethyl group; and q and R⁴ are as defined previously) followed where necessary by hydrolysis.

The reaction may be performed in a solvent such as water or an organic solvent such as a nitrile e.g. acetonitrile or an alcohol, e.g. ethanol or an amide e.g. dimethylformamide in the presence of a base such as an alkali metal carbonate or hydroxide, e.g. sodium, potassium or caesium carbonate, or sodium, potassium or lithium hydroxide, at an elevated temperature e.g. the reflux temperature.

Where appropriate, hydrolysis may be achieved using a base, such as described above, in a suitable solvent, for example sodium hydroxide in an alcohol such as ethanol.

In this reaction, the group Z may need to be in a protected state. Conventional protecting groups may be used, depending on the nature of Z, and may be removed using standard procedures, once the desired reaction has been effected.

Reagents D(CH₂)_{q}P(O)(OR¹³)R⁴ and D(CH₂)_{q}P(O)(OR¹³)₂ may be prepared by heating compounds of formulae P(OR¹³)₂R⁴ or P(OR¹³)₃ with a compound (CH₂)_{q}D₂.

In an alternative process, a compound of formula (1) [wherein in R¹, R² and/or R³ the group where present is an oxygen atom] may be prepared by reaction of a compound of formula (3), or a metal complex thereof with a phosphine R⁴P(OR¹³) in the presence of suitable aldehyde (for example formaldehyde or paraformaldehyde), followed by hydrolysis.

The reaction may be performed in an organic solvent, e.g. a nitrile, alcohol, or amide, or an ether such as tetrahydrofuran at an elevated temperature, for example the reflux temperature. Hydrolysis may be achieved using an acid, for example an inorganic acid such as hydrochloric acid, at an elevated temperature such as the reflux temperature.

Compounds of formula (1) may also be prepared by interconversion from other compounds of formula (1). Thus one functional group Z may be exchanged for another and, if desired a linker group L changed to another by appropriate manipulative reactions. For example, a compound of formula (1) where -L-Z is a group -L¹-NHCO-L²-Z (where -L¹-NHCO-L² represents the group L) may be prepared by reaction of a corresponding compound wherein -L-Z represents -L¹-NH₂ with a reagent R^{b}O-L²-Z (where R^{b} is for example am imide, such as succinimide, or a substituted phenyl group such as a p-nitrophenyl group) in the presence of a tertiary amine, such as diisopropylethylamine, is a solvent such as dimethylformamide.

Reagents of formula R^{b}O-L²-Z are either known compounds or may be obtained from known starting materials using methods analogous to those used for the preparation of the known compounds.

In another interconversion process, a compound of formula (1) wherein x where present is a sulphur atom may be prepared by reaction of a corresponding compound wherein X is an oxygen atom by reaction with a sulphide, for example phosphorous pentasulphide, at an elevated temperature.

It will be appreciated that where it is desired to prepare a compound of formula (1) in which one or two of R¹, R² and R³ are a hydrogen atom or a CO₂H group this may be achieved by first selectively N-protecting the compound of formula (3) or a precursor using an appropriate amine protecting group(s), for example a p-toluenesulphonyl group in accordance with conventional practice. Reaction of the N-protected compound (3) to introduce the required group -P(O)(XH)R⁴ using the methods described above followed by deprotection and further reaction with a group D(CH₂)_{q}H or D(CH₂)_{q}CO₂H (or an acid protected form thereof) using the reagents and conditions described previously for the introduction of the group -P(O)(XH)R4 then yields the desired compound in which one or two of R¹, R² and R³ are -H or CO₂H.

Where metal complexes of compounds of formulae (1) or (2) are required (or any other suitable macrocyclic intermediate described herein) these may be prepared by treating the compound with a metal salt (for example a metal halide e.g. a chloride, or a nitrate, acetate, carbonate or sulphate) or a metal oxide in an appropriate solvent for example an aqueous or non aqueous solvent, (e.g. acetonitrile, acetone, propylene carbonate, dimethylformamide or dimethylsulphoxide) at any suitable temperature from 0°C to 100°C such as 10°C to 80°C e.g. around 60°C.

A conjugate compound of formula (2) or a metal complex thereof may be prepared by reaction of a corresponding compound of formula (1) or a metal complex thereof with an antibody Ab (as previously defined).

The reaction may be performed in a suitable solvent, for example an aqueous solvent such as a phosphate buffer, at an appropriate temperature, for example at 0°C-30°C, especially 0°-10°C e.g. 4°C.

The antibody Ab may be obtained using procedures well known in the art. If desired, before the coupling reaction, the antibody may first be treated to yield appropriate groups for reaction with the compound of formula (1). Thus for example the antibody may be subjected to oxidation, for example periodate oxidation to yield aldehyde groups, or, in particular, may be treated with a reagent [e.g. Traut's reagent (2-iminothiolane)] using standard procedures to generate free sulphydryl groups in the molecule.

Salts of compounds of formulae (1) or (2) and their metal complexes may be prepared by conventional means, for example by reaction of a compound of formulae (1) or (2) with an appropriate base or acid in a suitable aqueous or non-aqueous solvent as described above, at any suitable temperature from 0°C to 100°C.

Intermediates of formula (3) may be prepared by the methods described in International Patent Specification Publication No. WO-A-8901475.

The invention is illustrated by the following Examples:

### Example 1

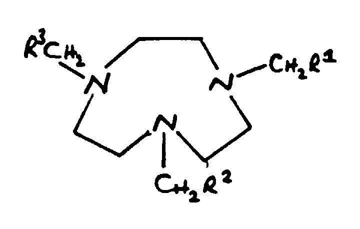

(a) R¹, R², R³ is each P(O)(OCH₂CH₃)CH₃
(b) R¹, R², R³ is each P(O) (OH)CH₃
(c) Indium complex of (b)
(d) Gallium complex of (b)

(a) To a solution of 1,4,7-triazacyclononane (90mg) and paraformaldelyde (0.10g) in dry tetrahydrofuran (5ml) was added methyl diethoxyphosphine (0.5g) and the solution was boiled under reflux (under N₂) for 48h. After removal of solvent, the residue was chromatographed on neutral alumina (eluant 0 → 5% methanol/CH₂Cl₂) to yield the desired triester product as a pale-yellow oil, Rf = 0.51 (3% methanol- CH₂Cl₂). ∂p (CD₂Cl₂) 54.2. m/e (DCI, CH₂Cl₂) 491, 490 [M⁺+1] 382, 289.
(b) The triester from (a)(50mg) was treated with hydrochloric acid (6M, 2ml) and was heated to 110° for 18h. Removal of solvent yielded the desired triacid as a glassy solid m/e (FAB, m-nitrobenzylalcohol) 406 (M⁺+1). ∂p (H₂O, pH1) 42.5. ∂_{H} (D₂O) 3.32 (18H, mult., CH₂CH₂N, + CH₂P), 1.42 (9H, d, J=14, P-CH₃).
   Admixture of equimolar quantities of the triacid prepared in (b) and either a solution of indium nitrate in dilute nitric acid (pH1) or gallium nitrate in nitric acid (pH1) yielded solutions of the corresponding complexes quantitatively with the following characteristics:
(c) Indium complex : ∂p (H₂O, pH1) + 38.3 - invariant over 4 weeks at pH1
(d) Gallium complex : ∂p (H₂O, pH1) + 40.5; ∂ Ga(H₂O, pH1) +137 ppm (invariant pH0 for 1 week); m/e (DCI methanol) = 474, 472 (M⁺ + 1).

### Example 2

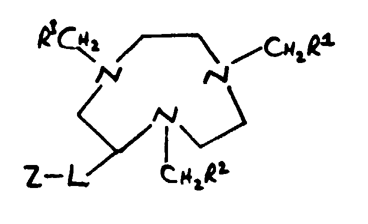

(a) R¹, R², R³ is each P(O)(OCH₂CH₃)CH₃; L-Z is -(CH₂)₄NHCO-phenyl
(b) R¹, R², R³ is each P(O)(OH)CH₃; L-Z is -(CH₂)₄NH₂.
(c) R¹, R², R³ is each P(O)(OH)CH₃; L-Z is -(CH₂)₄NHCO(CH₂)₂CO₂-Ph where Ph is 4-nitrophenyl.

(a) To a solution of 2-(4-N-benzamidyl)butyl-1,4,7, -triazacyclononane [prepared as described in International Patent Specification WO-A-8901475] (0.39g) in dry tetrahydrofuran (10ml) was added methyldiethoxyphosphine (0.66g) and paraformaldehyde (0.18g) and the mixture was heated to reflux for 18h with removal of water (Soxhlet: 4A molecular sieves). After filtration and removal of solvent, the residue was purified by chromatography on neutral alumina (0 → 3% methanol in CH₂Cl₂) to yield the desired tri-ester product as a colourless oil (0.26g). m/e (d.c.i.) 665 (M⁺+1), 557, 437, 126, 109. δ_{C} (CDCl₃) 167..4 (carbonyl); 134.75, 130.67, 121.86, 127.10 (aryl); 63.48 (CH₂O); 59.87, 59.03, 58.2, 58.15, 57.9, 57.8, 57.0, 53.1, 53.0, 52.7, (CH₂N); 39.4, 39.35 (CH₂NHCO, diastereoisomers); 28.6 (br.s, CH₂C); 24.0, 23.9 (CH₂C); 16.40, 16.36 (CH₃CH₂O: diastereomers); 13.22 (d, J_{CP}89HZ, major), 12.95, 12.80 (d+d, J_{CP}=90, 91Hz, CH₃P minor diastereoisomers) ν max (film) 3405, 3200 (NH); 2960, 2915, 2825 (CH); 1640 (NHCO,s); 1205 (vs); 1035 (vs), 950 (s) cm⁻¹. δ_{H} (CDCl₃) 7.91 (2H, dd, ortho arom.), 7.85 (1H, br. t, NHCO), 7.45-7.39 (3H, para + meta arom CH), 4.02 (6H, mult, CH₂O), 3.50-2.60 (19H, mult, CHN+CH₂N) 1.63 (2H, mult, CH₂CH₂NHCO) 1.55-1.42 (4H, mult, CH₂C), 1.30 (9H, mult, CH₃P), 1.20 (9H, t+t+t, CH₃CH₂O, diastereomers)
(b) A solution of the triester (0.13g), prepared in (a) in hydrochloric acid (6M, 5ml) was heated to reflux for 24h. After cooling, washing with ether (3 x 2ml), dichloromethane (2 x 5ml) and evaporation under high vacuum (0.01mm Hg, 18h), a colourless glass was obtained of the tetrahydrochloride salt of the corresponding triacid in which L-Z is (CH₂)₄NH₂. m/e (F.a.b.; glycerol): 477 (M⁺).
(c) To a solution of the triacid prepared in (a) (0.048g) in dry DMSO (0.5ml) was added N-methylmorpholine (90mg) and di-(p-nitrophenyl)-succinate (54mg) and the mixture was stirred for 18h at 20°C, monitoring by HPLC (Dynamax C18 60A, 21.4mm column: A=0.1% trifluoroacetic acid -H₂O), C=0.1% trifluoroacetic acid -CH₃CN: t=0 A=95%, C=5%; t=20 min A=5%, C=95%; flow = 10ml min⁻¹) Rₜ=12 mm. Purification by HPLC afforded the desired tri-acid as a colourless solid (25mg). m/e (F.a.b., glycerol) 699 (M⁺). δ_{H}(D₂O) 8.30 (2H, ortho Ar), 7.34 (2H, ortho Ar), 4.0 2.5 (23H, mult, CH₂CO+CH₂N), 1.8-1.5 (6H, mult, CH₂C), 1.25 (d+d+d, 9H, CH₃P).
   Reaction of the compound of part (c) with gallium nitrate or indium nitrate as described in Example 1 yielded the corresponding gallium or indium complexes.

## Claims (Claims for the following Contracting State(s): AT, BE, CH, LI, DE, DK, FR, GB, IT, LU, NL, SE)

1. A compound of formula (1): 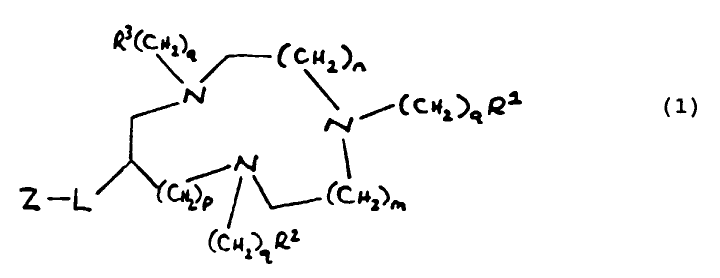 wherein
m and n, which may be the same or different, is each zero or an integer 1, 2 or 3;
p is zero or an integer 1 or 2;
q is zero or an integer from 1 to 6 inclusive;
R¹, R² and R³, which may be the same or different, is each a hydrogen atom or a group -CO₂H or -P(O) (XH)R⁴ (where X is an oxygen or sulphur atom and R⁴ is a hydrogen atom or an alkyl or alkoxy group), with the proviso that at least one of R¹, R² and R³ is a -P(O) (XH)R⁴ group;
L is a covalent bond or a linker group;
Z is a hydrogen atom or a reactive functional group capable of reacting with a thiol, amino, carboxyl, hydroxyl, aldehyde, aromatic or heteroaromatic group;
and metal complexes and/or salts thereof.

2. A compound according to claim 1 where R¹, R² and R³ are the same and is each a group -P(O)(XH)R⁴.

3. A compound according to claim 2 wherein R¹, R² and R³ is each a group -P(O) (OH)CH₃.

4. A compound according to any of the preceding claims wherein q is an integer 1.

5. A compound according to any of the preceding claims wherein L is a covalent bond and Z is a hydrogen atom.

6. A compound according to any one of claims 1 to 4 wherein L is an optionally substituted aliphatic hydrocarbyl chain, optionally interrupted by one or more heteroatoms selected from -O- or -S- or by one or more -N(R⁵)-, -N(R⁵) CO-, cycloaliphatic, aromatic, or heteroaromatic groups,
wherein the optional substituents comprise halogen atoms, or groups selected from C₁₋₆alkoxy, hydroxy, nitro, -N(R⁶)(R⁷) [where R⁶ is a hydrogen atom or a C₁₋₆alkyl group and R⁷ is a C₁₋₆alkyl group], or a group of formula -(CH₂)ₙCON(R⁸)(R⁹) [where n is zero or an integer 1 to 4 inclusive, R⁸ is a hydrogen atom or a C₁₋₆alkyl group and R⁹ is a C₁₋₆ alkyl group optionally substituted by one or more halogen atoms, or nitro, amino or hydroxy groups].

7. A compound according to claim 1 of formula (1a): 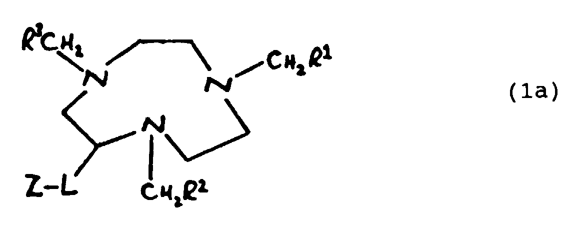 wherein
R¹, R², and R³, which may be the same or different, is each a hydrogen atom or a group -CO₂H or -P(O)(XH)R⁴ (where X is an oxygen or sulphur atom and R⁴ is a hydrogen atom or an alkyl or alkoxy group), with the proviso that at least one of R¹, R² and R³ is a -P(O)(XH)R⁴ group;
L is a covalent bond or a linker group;
Z is a hydrogen atom or a reactive functional group capable of reacting with a thiol, amino, carboxyl, hydroxyl, aldehyde, aromatic or heteroaromatic group;
and metal complexes and/or salts thereof.

8. A compound according to claim 7 wherein R¹, R² and R³ is each a group -P(O)(OH)CH₃.

9. A compound according to claim 7 or 8 wherein L is an optionally substituted aliphatic hydrocarbyl chain, optionally interrupted by one or more heteroatoms selected from -O- or -S- or by one or more -N(R⁵)-, -N(R⁵)CO-, cycloaliphatic, aromatic, or heteroaromatic groups and Z is any group capable of reacting with a thiol, amino, carboxyl, hydroxyl, aldehyde, aromatic or heteroaromatic group, wherein the optional substituents comprise halogen atoms, or groups selected from C₁₋₆alkoxy, hydroxy, nitro, -N(R⁶)(R⁷) [where R⁶ is a hydrogen atom or a C₁₋₆alkyl group and R⁷ is a C₁₋₆alkyl group], or a group of formula -(CH₂)ₙCON(R⁸)(R⁹) [where n is zero or an integer 1 to 4 inclusive, R⁸ is a hydrogen atom or a C₁₋₆alkyl group and R⁹ is a C₁₋₆ alkyl group optionally substituted by one or more halogen atoms, or nitro, amino or hydroxy groups].

10. A compound according to claim 7 or 8 wherein L is a covalent bond and Z is a hydrogen atom.

11. An indium or gallium complex of a compound according to any of the preceding claims.

12. A gallium complex of a compound according to claim 10.

13. A conjugate compound of formula (2): 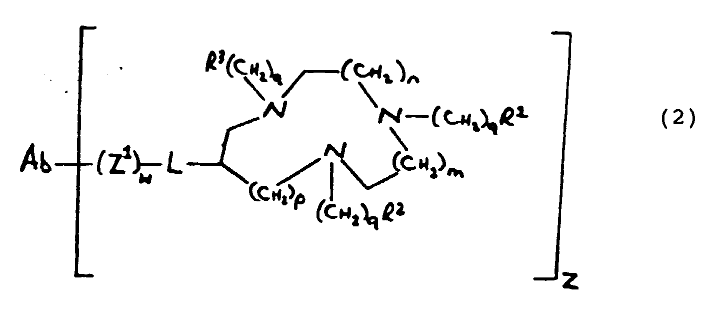 wherein
m and n, which may be the same or different, is each zero or an integer 1, 2 or 3;
p is zero or an integer 1 or 2;
q is zero or an integer from 1 to 6 inclusive;
R¹, R² and R³, which may be the same or different, is each a hydrogen atom or a group -CO₂H or -P(O)(XH)R⁴ (where X is an oxygen or sulphur atom and R⁴ is a hydrogen atom or an alkyl or alkoxy group), with the proviso that at least one of R¹, R² and R³ is a -P(O)(XH)R⁴ group;
L is a covalent bond or a linker group;
Z¹ is the residue of a reactive functional group capable of reacting with a thiol, amino, carboxyl, hydroxyl, aldehyde, aromatic or heteroaromatic group;
w is zero or an integer 1;
z is an integer 1 or more;
Ab is an antibody;
and metal complexes and/or salts thereof.

14. A conjugate compound according to claim 13 wherein R¹, R², R³, q and L are as defined in any of claims 2, 3, 4 and 6.

15. A conjugate compound according to claim 13 of formula (2a): 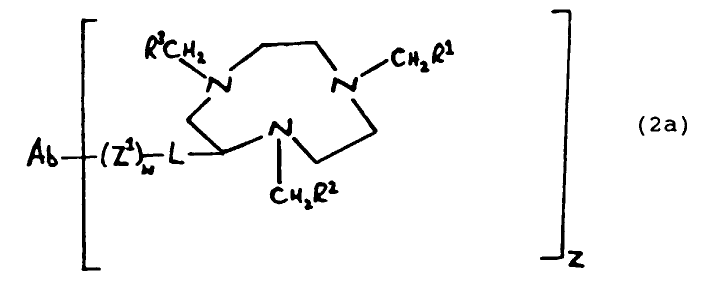 wherein
R¹, R², and R³, which may be the same or different, is each a hydrogen atom or a group -CO₂H or -P(O)(XH)R⁴ (where X is an oxygen or sulphur atom and R⁴ is a hydrogen atom or an alkyl or alkoxy group), with the proviso that at least one of R¹, R² and R³ is a -P(O)(XH)R⁴ group;
L is a covalent bond or a linker group;
Z¹ is the residue of a reactive functional group capable of reacting with a thiol, amino, carboxyl, hydroxyl, aldehyde, aromatic or heteroaromatic group;
w is zero or an integer 1;
z is an integer 1 or more;
Ab is an antibody;
and metal complexes and/or salts thereof.

16. A conjugate compound according to claim 15 wherein R¹, R² and R³ is each a group -P(O)(OH)CH₃.

17. A conjugate compound according to claim 15 or 16 wherein L is an optionally substituted aliphatic hydrocarbyl chain, optionally interrupted by one or more heteroatoms selected from -O- or -S- or by one or more -N(R⁵)-, -N(R⁵)CO-, cycloaliphatic, aromatic, or heteroaromatic groups and Z¹ is the residue of any group capable of reacting with a thiol, amino, carboxyl, aldehyde, aromatic or heteroaromatic group, wherein the optional substituents comprise halogen atoms, or groups selected from C₁₋₆alkoxy, hydroxy, nitro, -N(R⁶)(R⁷) [where R⁶ is a hydrogen atom or a C₁₋₆alkyl group and R⁷ is a C₁₋₆alkyl group], or a group of formula -(CH₂)ₙCON(R⁸)(R⁹) [where n is zero or an integer 1 to 4 inclusive, R⁸ is a hydrogen atom or a C₁₋₆alkyl group and R⁹ is a C₁₋₆ alkyl group optionally substituted by one or more halogen atoms, or nitro, amino or hydroxy groups].

18. An indium or gallium complex of a conjugate compound according to any one of claims 13 to 17.

## Claims (Claims for the following Contracting State(s): ES, GR)

1. A process for the preparation of a compound of formula (1): 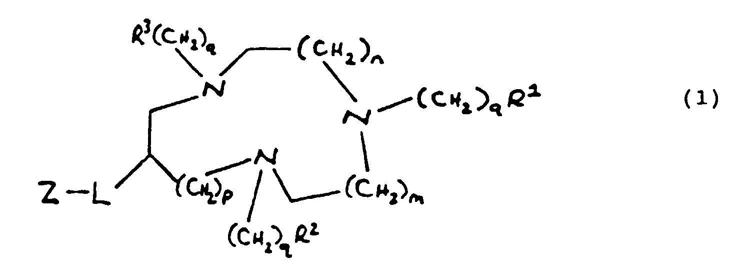 wherein
m and n, which may be the same or different, is each zero or an integer 1, 2 or 3;
p is zero or an integer 1 or 2;
q is zero or an integer from 1 to 6 inclusive;
R¹, R² and R³, which may be the same or different, is each a hydrogen atom or a group -CO₂H or -P(O)(XH)R⁴ (where X is an oxygen or sulphur atom and R⁴ is a hydrogen atom or an alkyl or alkoxy group), with the proviso that at least one of R¹, R² and R³ is a -P(O)(XH)R⁴ group;
L is a covalent bond or a linker group;
Z is a hydrogen atom or a reactive functional group capable of reacting with a thiol, amino, carboxyl, hydroxyl, aldehyde, aromatic or heteroaromatic group;
and metal complexes and/or salts thereof;
the process comprising the step of reacting a compound of formula (3) 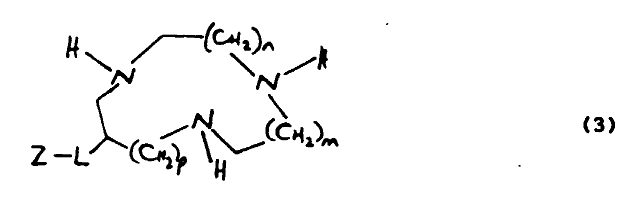 or a metal complex thereof with a reagent D(CH₂)_{q}P(O)(OH)R⁴, D(CH₂)_{q}H or D(CH₂)_{q}CO₂H (or acid protected forms thereof) where D is a displaceable group.

2. A process according to claim 1 where R¹, R² and R³ are the same and is each a group -P(O)(XH)R⁴.

3. A process according to claim 2 wherein R¹, R² and R³ is each a group -P(O)(OH)CH₃.

4. A process according to any of the preceding claims wherein q is an integer 1.

5. A process according to any of the preceding claims wherein L is a covalent bond and Z is a hydrogen atom.

6. A process according to any one of claims 1 to 4 wherein L is an optionally substituted aliphatic hydrocarbyl chain, optionally interrupted by one or more heteroatoms selected from -O- or -S- or by one or more -N(R⁵)-, -N(R⁵)CO-, cycloaliphatic, aromatic, or heteroaromatic groups,
wherein the optional substituents comprise halogen atoms, or groups selected from C₁₋₆alkoxy, hydroxy, nitro, -N(R⁶)(R⁷) [where R⁶ is a hydrogen atom or a C₁₋₆alkyl group and R⁷ is a C₁₋₆alkyl group], or a group of formula -(CH₂)ₙCON(R⁸)(R⁹) [where n is zero or an integer 1 to 4 inclusive, R⁸ is a hydrogen atom or a C₁₋₆alkyl group and R⁹ is a C₁₋₆ alkyl group optionally substituted by one or more halogen atoms, or nitro, amino or hydroxy groups].

7. A process according to claim 1 of formula (1a): 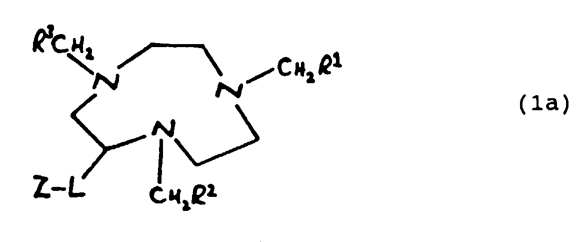 wherein
R¹, R², and R³, which may be the same or different, is each a hydrogen atom or a group -CO₂H or -P(O)(XH)R⁴ (where X is an oxygen or sulphur atom and R⁴ is a hydrogen atom or an alkyl or alkoxy group), with the proviso that at least one of R¹, R² and R³ is a -P(O)(XH)R⁴ group;
L is a covalent bond or a linker group;
Z is a hydrogen atom or a reactive functional group capable of reacting with a thiol, amino, carboxyl, hydroxyl, aldehyde, aromatic or heteroaromatic group;
and metal complexes and/or salts thereof.

8. A process according to claim 7 wherein R¹, R² and R³ is each a group -P(O)(OH)CH₃.

9. A process according to claim 7 or 8 wherein L is an optionally substituted aliphatic hydrocarbyl chain, optionally interrupted by one or more heteroatoms selected from -O- or -S- or by one or more -N(R⁵)-, -N(R⁵)CO-, cycloaliphatic, aromatic, or heteroaromatic groups and Z is any group capable of reacting with a thiol, amino, carboxyl, hydroxyl, aldehyde, aromatic or heteroaromatic group, wherein the optional substituents comprise halogen atoms, or groups selected from C₁₋₆alkoxy, hydroxy, nitro, -N(R⁶)(R⁷) [where R⁶ is a hydrogen atom or a C₁₋₆alkyl group and R⁷ is a C₁₋₆alkyl group], or a group of formula -(CH₂)ₙCON(R⁸)(R⁹) [where n is zero or an integer 1 to 4 inclusive, R⁸ is a hydrogen atom or a C₁₋₆alkyl group and R⁹ is a C₁₋₆ alkyl group optionally substituted by one or more halogen atoms, or nitro, amino or hydroxy groups].

10. A process according to claim 7 or 8 wherein L is a covalent bond and Z is a hydrogen atom.

11. A process according to any of the preceding claims comprising the step of complexation with indium or gallium.

12. A process according to claim 11 comprising the step of complexation with gallium.

13. A process for the preparation of a conjugate compound of formula (2): 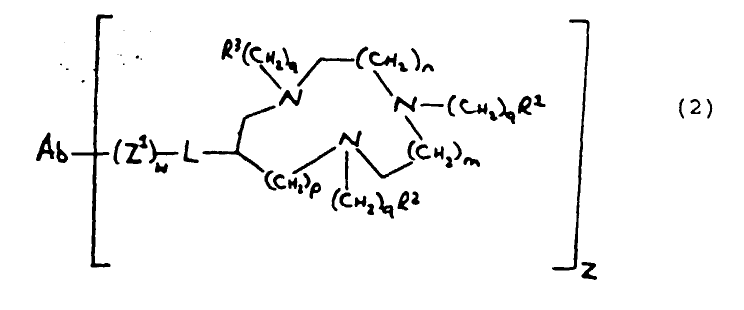 wherein
m and n, which may be the same or different, is each zero or an integer 1, 2 or 3;
p is zero or an integer 1 or 2;
q is zero or an integer from 1 to 6 inclusive;
R¹, R² and R³, which may be the same or different, is each a hydrogen atom or a group -CO₂H or -P(O)(XH)R⁴ (where X is an oxygen or sulphur atom and R⁴ is a hydrogen atom or an alkyl or alkoxy group), with the proviso that at least one of R¹, R² and R³ is a -P(O)(XH)R⁴ group;
L is a covalent bond or a linker group;
Z¹ is the residue of a reactive functional group capable of reacting with a thiol, amino, carboxyl, hydroxyl, aldehyde, aromatic or heteroaromatic group;
w is zero or an integer 1;
z is an integer 1 or more;
Ab is an antibody;
and metal complexes and/or salts thereof;
the process comprising the step of reacting a compound of formula (1) 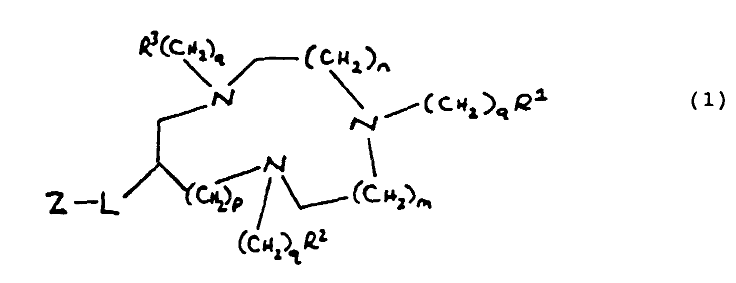 or a metal complex thereof with an antibody.

14. A process according to claim 13 wherein R¹, R², R³, q and L are as defined in any of claims 2, 3, 4 and 6.

15. A process according to claim 13 for the preparation of a compound of formula (2a): 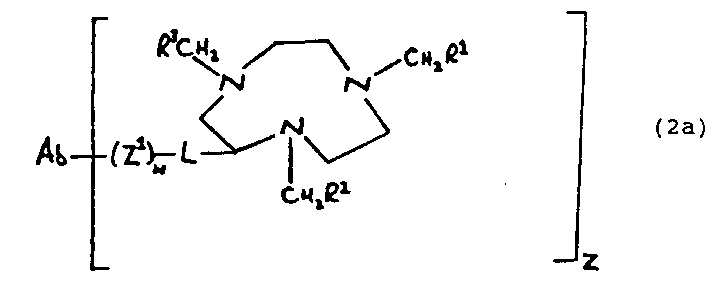 wherein
R¹, R², and R³, which may be the same or different, is each a hydrogen atom or a group -CO₂H or -P(O)(XH)R⁴ (where X is an oxygen or sulphur atom and R⁴ is a hydrogen atom or an alkyl or alkoxy group), with the proviso that at least one of R¹, R² and R³ is a -P(O)(XH)R⁴ group;
L is a covalent bond or a linker group;
Z¹ is the residue of a reactive functional group capable of reacting with a thiol, amino, carboxyl, hydroxyl, aldehyde, aromatic or heteroaromatic group;
w is zero or an integer 1;
z is an integer 1 or more;
Ab is an antibody;
and metal complexes and/or salts thereof.

16. A process according to claim 15 wherein R¹, R² and R³ is each a group -P(O)(OH)CH₃.

17. A process according to claim 15 or 16 wherein L is an optionally substituted aliphatic hydrocarbyl chain, optionally interrupted by one or more heteroatoms selected from -O- or -S- or by one or more -N(R⁵)-, -N(R⁵)CO-, cycloaliphatic, aromatic, or heteroaromatic groups and Z¹ is the residue of any group capable of reacting with a thiol, amino, carboxyl, aldehyde, aromatic or heteroaromatic group, wherein the optional substituents comprise halogen atoms, or groups selected from C₁₋₆alkoxy, hydroxy, nitro, -N(R⁶)(R⁷) [where R⁶ is a hydrogen atom or a C₁₋₆alkyl group and R⁷ is a C₁₋₆alkyl group], or a group of formula -(CH₂)ₙCON(R⁸)(R⁹) [where n is zero or an integer 1 to 4 inclusive, R⁸ is a hydrogen atom or a C₁₋₆alkyl group and R⁹ is a C₁₋₆ alkyl group optionally substituted by one or more halogen atoms, or nitro, amino or hydroxy groups].

18. A process according to any one of claims 13 to 17 comprising the step of complexation with indium or gallium.

## Patentansprüche (Patentansprüche für folgende(n) Vertragsstaat(en): AT, BE, CH, LI, DE, DK, FR, GB, IT, LU, NL, SE)

1. Verbindung der Formel (1): 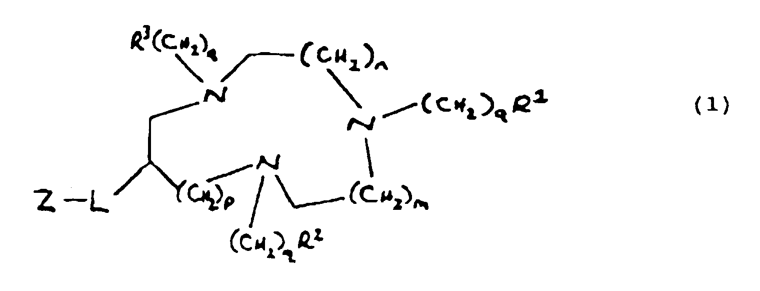 wobei
m und n, die gleich oder unterschiedlich sein können, jeweils Null oder eine Zahl 1, 2 oder 3 sind,
p Null oder eine Zahl 1 oder 2 ist,
q Null oder eine Zahl von 1 bis einschließlich 6 ist,
R¹, R² und R³, die gleich oder unterschiedlich sein können, jeweils ein Wasserstoffatom oder eine -CO₂H -Gruppe oder eine -P(O)(XH)R⁴ -Gruppe (worin X ein Sauerstoff- oder Schwefelatom ist und R⁴ ein Wasserstoffatom oder ein Alkyl- oder ein Alkoxyrest ist) sind, mit der Maßgabe, daß mindestens einer der Reste R¹, R² und R³ eine -P(O)(XH)R⁴ -Gruppe ist,
L eine kovalente Bindung oder eine Linkergruppe ist,
Z ein Wasserstoffatom oder eine reaktive funktionelle Gruppe, befähigt zur Reaktion mit einer Thiol-, Amino-, Carboxyl-, Hydroxyl-, Aldehyd-, aromatischen oder heteroaromatischen Gruppe, ist,
und Metallkomplexe und/oder Salze davon.

2. Verbindung nach Anspruch 1, wobei R¹, R² und R³ gleich sind und jeweils eine -P(O)(XH)R⁴ -Gruppe bedeuten.

3. Verbindung nach Anspruch 2, wobei R¹, R² und R³ jeweils eine -P(O)(OH)CH₃ -Gruppe bedeuten.

4. Verbindung nach einem der vorstehenden Ansprüche, wobei q die Zahl 1 ist.

5. Verbindung nach einem der vorstehenden Ansprüche, wobei L eine kovalente Bindung ist und Z ein Wasserstoffatom ist.

6. Verbindung nach einem der Ansprüche 1 bis 4, wobei L eine gegebenenfalls substituierte, aliphatische Kohlenwasserstoffkette ist, gegebenenfalls durch ein oder mehrere Heteroatome, ausgewählt aus -O- oder -S-, oder durch ein oder mehrere -N(R⁵)-, -N(R⁵)CO-, cycloaliphatische, aromatische oder heteroaromatische Gruppen unterbrochen, ist,
wobei die fakultativen Substituenten Halogenatome oder Gruppen, ausgewählt aus C₁₋₆Alkoxy, Hydroxy, Nitro, -N(R⁶)(R⁷) [worin R⁶ ein Wasserstoffatom ist oder ein C₁₋₆Alkylrest ist und R⁷ ein C₁₋₆Alkylrest ist], oder eine Gruppe der Formel -(CH₂)ₙCON(R⁸)(R⁹) [worin n Null ist oder eine Zahl von 1 bis einschließlich 4 ist, R⁸ ein Wasserstoffatom oder ein C₁₋₆Alkylrest ist und R⁹ ein C₁₋₆Alkylrest, gegebenenfalls substituiert durch ein oder mehrere Halogenatome, oder eine Nitro-, Amino- oder Hydroxygruppe ist] umfassen.

7. Verbindung nach Anspruch 1 der Formel (1a): 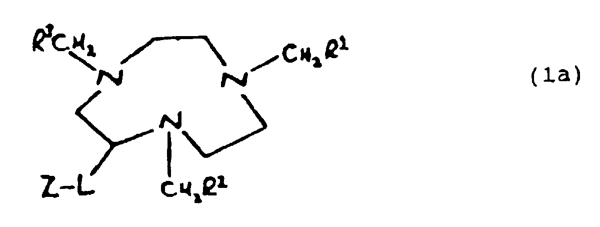 wobei
R¹, R² und R³, die gleich oder unterschiedlich sein können, jeweils ein Wasserstoffatom oder eine -CO₂H- oder -P(O)(XH)R⁴ -Gruppe (worin X ein Sauerstoff- oder Schwefelatom ist und R⁴ ein Wasserstoffatom oder ein Alkyl- oder Alkoxyrest ist) sind, mit der Maßgabe, daß mindestens einer der Reste R¹, R² und R³ eine -P(O)(XH)R⁴ -Gruppe ist,
L ein kovalente Bindung oder eine Linkergruppe ist,
Z ein Wasserstoffatom oder eine reaktive funktionelle Gruppe, befähigt zur Reaktion mit einer Thiol-, Amino-, Carboxyl-, Hydroxyl-, Aldehyd-, aromatischen oder heteroaromatischen Gruppe, ist,
und Metallkomplexe und/oder Salze davon.

8. Verbindung nach Anspruch 7, wobei R¹, R² und R³ jeweils eine -P(O)(OH)CH₃-Gruppe sind.

9. Verbindung nach Anspruch 7 oder 8, wobei L eine gegebenenfalls substituierte, aliphatische Kohlenwasserstoffkette, gegebenenfalls durch ein oder mehrere Heteroatome, ausgewählt aus -O- oder -S-, oder durch ein oder mehrere -N(R⁵)-, -N(R⁵)CO-, cycloaliphatische, aromatische oder heteroaromatische Gruppen unterbrochen, ist und Z eine Gruppe, befähigt zur Reaktion mit einer Thiol-, Amino-, Carboxyl-, Hydroxyl-, Aldehyd-, aromatischen oder heteroaromatischen Gruppe, ist,
wobei die fakultativen Substituenten Halogenatome oder Gruppen, ausgewählt aus C₁₋₆Alkoxy, Hydroxy, Nitro, -N(R⁶)(R⁷) [worin R⁶ ein Wasserstoffatom oder eine C₁₋₆Alkylrest ist und R⁷ ein C₁₋₆Alkylrest ist], oder eine Gruppe der Formel -(CH₂)ₙCFN(R⁸)(R⁹) [worin n Null oder eine Zahl von 1 bis einschließlich 4 ist, R⁸ ein Wasserstoffatom oder ein C₁₋₆Alkylrest ist und R⁹ ein C₁₋₆Alkylrest, gegebenenfalls substituiert durch ein oder mehrere Halogenatome oder Nitro-, Amino- oder Hydroxygruppen ist] umfassen.

10. Verbindung nach Anspruch 7 oder 8, wobei L eine kovalente Bindung ist und Z ein Wasserstoffatom ist.

11. Indium- oder Galliumkomplex einer Verbindung nach einem der vorstehenden Ansprüche.

12. Galliumkomplex einer Verbindung nach Anspruch 10.

13. Konjugierte Verbindung der Formel (2): 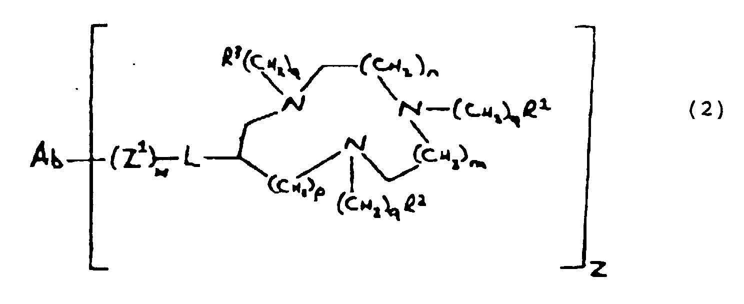 wobei
m und n, die gleich oder unterschiedlich sein können, jeweils Null oder die Zahl 1, 2 oder 3 sind,
p Null oder eine Zahl 1 oder 2 ist,
q Null oder eine Zahl von 1 bis einschließlich 6 ist,
R¹, R² und R³, die gleich oder unterschiedlich sein können, jeweils ein Wasserstoffatom oder eine -CO₂H- oder -P(O)(XH)R⁴-Gruppe (worin X ein Sauerstoff- oder Schwefelatom ist und R⁴ ein Wasserstoffatom oder ein Alkyl- oder Alkoxyrest ist) sind, mit der Maßgabe, daß mindestens einer der Reste R¹, R² und R³ eine -P(O)(XH)R⁴-Gruppe ist,
L ein kovalente Bindung oder eine Linkergruppe ist,
Z¹ der Rest einer reaktiven funktionellen Gruppe, befähigt zur Reaktion mit einer Thiol-, Amino-, Carboxyl-, Hydroxyl-, Aldehyd-, aromatischen oder heteroaromatischen Gruppe, ist,
w Null oder die Zahl 1 ist,
z eine Zahl 1 oder mehr ist,
Ab ein Antikörper ist,
und Metallkomplexe und/oder Salze davon.

14. Konjugierte Verbindung nach Anspruch 13, wobei R¹, R², R³, q und L wie in einem der Ansprüche 2, 3, 4 und 6 definiert sind.

15. Konjugierte Verbindung nach Anspruch 13 der Formel (2a): 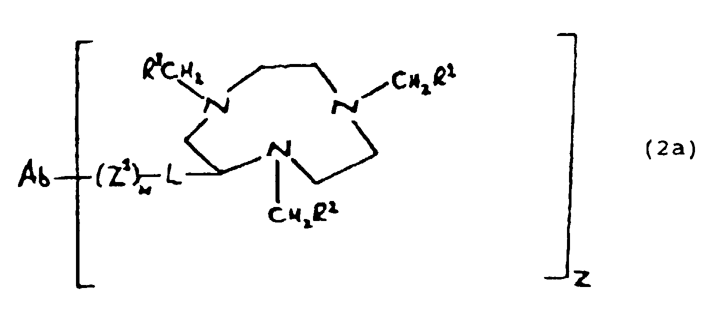 wobei
R¹, R² und R³, die gleich oder unterschiedlich sein können, jeweils ein Wasserstoffatom oder eine -CO₂H- oder -P(O)(XH)R⁴-Gruppe (worin X ein Sauerstoff- oder Schwefelatom ist und R⁴ ein Wasserstoffatom oder ein Alkyl- oder Alkoxyrest ist), sind, mit der Maßgabe, daß mindestens einer der Reste R¹, R² und R³ eine -P(O)(XH)R⁴-Gruppe ist,
L eine kovalente Bindung oder eine Linkergruppe ist,
Z¹ der Rest einer reaktiven funktionellen Gruppe, befähigt zur Reaktion mit einer Thiol-, Amino-, Carboxyl-, Hydroxyl-, Aldehyd-, aromatischen oder heteroaromatischen Gruppe, ist,
w Null oder eine Zahl 1 ist,
z eine Zahl 1 oder mehr ist,
Ab ein Antikörper ist,
und Metallkomplexe und/oder Salze davon.

16. Konjugierte Verbindung nach Anspruch 15, wobei R¹, R² und R³ jeweils eine -P(O)(OH)CH₃-Gruppe sind.

17. Konjugierte Verbindung nach Anspruch 15 oder 16, wobei L eine gegebenenfalls substituierte, aliphatische Kohlenwasserstoffkette; gegebenenfalls durch ein oder mehrere Heteroatome, ausgewählt aus -O- oder -S-, oder durch eine oder mehrere -N(R⁵)-, -N(R⁵)CO-, cycloaliphatischen, aromatischen oder heteroaromatischen Gruppen unterbrochen, ist und Z¹ der Rest einer Gruppe, befähigt zur Reaktion mit einer Thiol-, Amino-, Carboxyl-, Aldehyd-, aromatischen oder heteroaromatischen Gruppe, ist, wobei die fakultativen Substituenten Halogenatome, oder Gruppen ausgewählt aus C₁₋₆Alkoxy, Hydroxy, Nitro, -N(R⁶)(R⁷) [worin R⁶ ein Wasserstoffatom oder ein C₁₋₆Alkylrest ist und R⁷ ein C₁₋₆Alkylrest ist], oder eine Gruppe der Formel -(CH₂)ₙCON(R⁸)(R⁹) [worin n Null oder eine Zahl von 1 bis einschließlich 4 ist, R⁸ ein Wasserstoffatom oder ein C₁₋₆Alkylrest ist und R⁹ ein C₁₋₆Alkylrest, gegebenenfalls substituiert durch ein oder mehrere Halogenatome, oder Nitro-, Amino- oder Hydroxygruppen ist] umfassen.

18. Indium- oder Galliumkomplex einer konjugierten Verbindung nach einem der Ansprüche 13 bis 17.

## Patentansprüche (Patentansprüche für folgende(n) Vertragsstaat(en): ES, GR)

1. Verfahren zur Herstellung einer Verbindung der Formel (1): 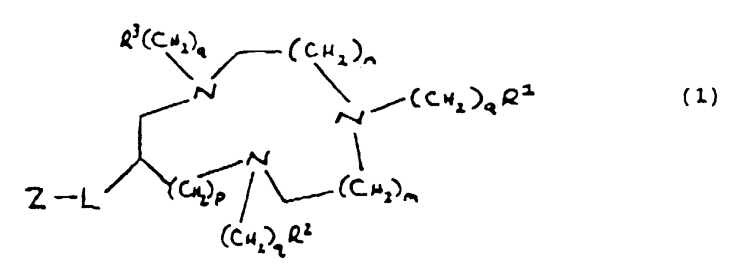 wobei
m und n, die gleich oder unterschiedlich sein können, jeweils Null oder eine Zahl 1, 2 oder 3 sind,
p Null oder eine Zahl 1 oder 2 ist,
q Null oder eine Zahl von 1 bis einschließlich 6 ist,
R¹, R² und R³, die gleich oder unterschiedlich sein können, jeweils ein Wasserstoffatom oder eine -CO₂H -Gruppe oder eine -P(O)(XH)R⁴-Gruppe (worin X ein Sauerstoff- oder Schwefelatom ist und R⁴ ein Wasserstoffatom oder ein Alkyl- oder ein Alkoxyrest ist) sind, mit der Maßgabe, daß mindestens einer der Reste R¹, R² und R³ eine -P(O)(XH)R⁴-Gruppe ist,
L eine kovalente Bindung oder eine Linkergruppe ist,
Z ein Wasserstoffatom oder eine reaktive funktionelle Gruppe, befähigt zur Reaktion mit einer Thiol-, Amino-, Carboxyl-, Hydroxyl-, Aldehyd-, aromatischen oder heteroaromatischen Gruppe, ist,
und Metallkomplexe und/oder Salze davon,
wobei das Verfahren den Schritt des Umsetzens einer Verbindung der Formel (3) 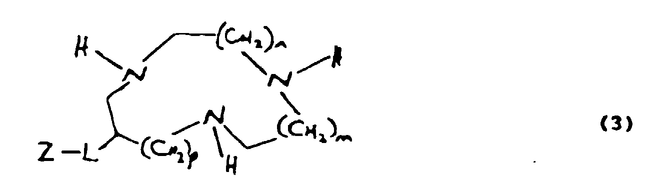 oder einem Metallkomplex davon mit einem Reagens D(CH₂)_{q}P(O)(OH)R⁴, D(CH₂)_{q}H oder D(CH₂)_{q}CO₂H (oder säuregeschützte Formen davon), wobei D eine verdrängbare Gruppe ist, umfaßt.

2. Verfahren nach Anspruch 1, wobei R¹, R² und R³ gleich sind und jeweils eine -P(O)(XH)R⁴ -Gruppe bedeuten.

3. Verfahren nach Anspruch 2, wobei R¹, R² und R³ jeweils eine -P(O)(OH)CH₃ -Gruppe bedeuten.

4. Verfahren nach einem der vorstehenden Ansprüche, wobei q die Zahl 1 ist.

5. Verfahren nach einem der vorstehenden Ansprüche, wobei L eine kovalente Bindung ist und Z ein Wasserstoffatom ist.

6. Verfahren nach einem der Ansprüche 1 bis 4, wobei L eine gegebenenfalls substituierte, aliphatische Kohlenwasserstoffkette ist, gegebenenfalls durch ein oder mehrere Heteroatome, ausgewählt aus -O- oder -S- oder durch ein oder mehrere -N(R⁵)-, -N(R⁵)CO-, cycloaliphatische, aromatische oder heteroaromatische Gruppen unterbrochen, ist,
wobei die fakultativen Substituenten Halogenatome oder Gruppen, ausgewählt aus C₁₋₆Alkoxy, Hydroxy, Nitro, -N(R⁶)(R⁷) [worin R⁶ ein Wasserstoffatom ist oder ein C₁₋₆Alkylrest ist und R⁷ ein C₁₋₆Alkylrest ist], oder eine Gruppe der Formel -(CH₂)ₙCON(R⁸)(R⁹) [worin n Null ist oder eine Zahl von 1 bis einschließlich 4 ist, R⁸ ein Wasserstoffatom oder ein C₁₋₆Alkylrest ist und R⁹ ein C₁₋₆Alkylrest, gegebenenfalls substituiert durch ein oder mehrere Halogenatome, oder eine Nitro-, Amino- oder Hydroxygruppe ist] umfassen.

7. Verfahren nach Anspruch 1 der Formel (1a): 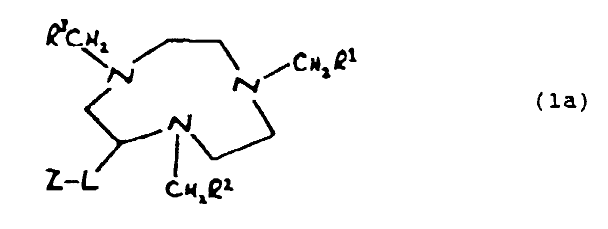 wobei
R¹, R² und R³, die gleich oder unterschiedlich sein können, jeweils ein Wasserstoffatom oder eine -CO₂H- oder -P(O)(XH)R⁴ -Gruppe (worin X ein Sauerstoff- oder Schwefelatom ist und R⁴ ein Wasserstoffatom oder ein Alkyl- oder Alkoxyrest ist) sind, mit der Maßgabe, daß mindestens einer der Reste R¹, R² und R³ eine -P(O)(XH)R⁴ -Gruppe ist,
L ein kovalente Bindung oder eine Linkergruppe ist,
Z ein Wasserstoffatom oder eine reaktive funktionelle Gruppe, befähigt zur Reaktion mit einer Thiol-, Amino-, Carboxyl-, Hydroxyl-, Aldehyd-, aromatischen oder heteroaromatischen Gruppe, ist,
und Metallkomplexe und/oder Salze davon.

8. Verfahren nach Anspruch 7, wobei R¹, R² und R³ jeweils eine -P(O)(OH)CH₃-Gruppe sind.

9. Verfahren nach Anspruch 7 oder 8, wobei L eine gegebenenfalls substituierte, aliphatische Kohlenwasserstoffkette, gegebenenfalls durch ein oder mehrere Heteroatome, ausgewählt aus -O- oder -S-, oder durch ein oder mehrere -N(R⁵)-, -N(R⁵)CO-, cycloaliphatische, aromatische oder heteroaromatische Gruppen unterbrochen, ist und Z eine Gruppe, befähigt zur Reaktion mit einer Thiol-, Amino-, Carboxyl-, Hydroxyl-, Aldehyd-, aromatischen oder heteroaromatischen Gruppe, ist,
wobei die fakultativen Substituenten Halogenatome oder Gruppen, ausgewählt aus C₁₋₆Alkoxy, Hydroxy, Nitro, -N(R⁶)(R⁷) [worin R⁶ ein Wasserstoffatom oder eine C₁₋₆Alkylrest ist und R⁷ ein C₁₋₆Alkylrest ist], oder eine Gruppe der Formel -(CH₂)ₙCON(R⁸)(R⁹) [worin n Null oder eine Zahl von 1 bis einschließlich 4 ist, R⁸ ein Wasserstoffatom oder ein C₁₋₆Alkylrest ist und R⁹ ein C₁₋₆Alkylrest, gegebenenfalls substituiert durch ein oder mehrere Halogenatome oder Nitro-, Amino- oder Hydroxygruppen ist] umfassen.

10. Verfahren nach Anspruch 7 oder 8, wobei L eine kovalente Bindung ist und Z ein Wasserstoffatom ist.

11. Verfahren nach einem der vorstehenden Ansprüche, umfassend den Schritt der Komplexierung mit Indium oder Gallium.

12. Verfahren nach Anspruch 11, umfassend den Schritt der Komplexierung mit Gallium.

13. Verfahren zur Herstellung einer konjugierten Verbindung der Formel (2): 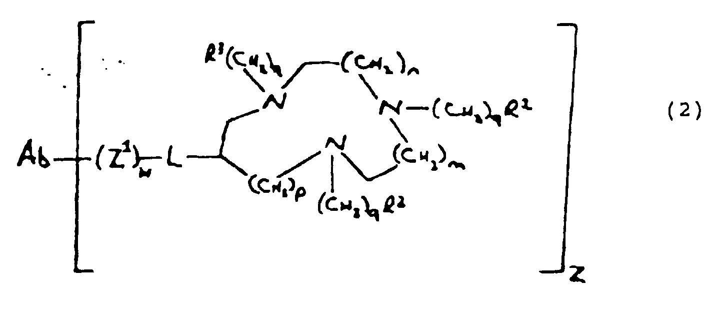 wobei
m und n, die gleich oder unterschiedlich sein können, jeweils Null oder die Zahl 1, 2 oder 3 sind,
p Null oder eine Zahl 1 oder 2 ist,
q Null oder eine Zahl von 1 bis einschließlich 6 ist,
R¹, R² und R³, die gleich oder unterschiedlich sein können, jeweils ein Wasserstoffatom oder eine -CO₂H- oder -P(O)(XH)R⁴-Gruppe (worin X ein Sauerstoff- oder Schwefelatom ist und R⁴ ein Wasserstoffatom oder ein Alkyl- oder Alkoxyrest ist) sind, mit der Maßgabe, daß mindestens einer der Reste R¹, R² und R³ eine -P(O)(XH)R⁴-Gruppe ist,
L ein kovalente Bindung oder eine Linkergruppe ist,
Z¹ der Rest einer reaktiven funktionellen Gruppe, befähigt zur Reaktion mit einer Thiol-, Amino-, Carboxyl-, Hydroxyl-, Aldehyd-, aromatischen oder heteroaromatischen Gruppe, ist,
w Null oder eine Zahl 1 ist,
z eine Zahl 1 oder mehr ist,
Ab ein Antikörper ist,
und Metallkomplexe und/oder Salze davon,
wobei das Verfahren den Schritt des Umsetzens einer Verbindung der Formel (1) 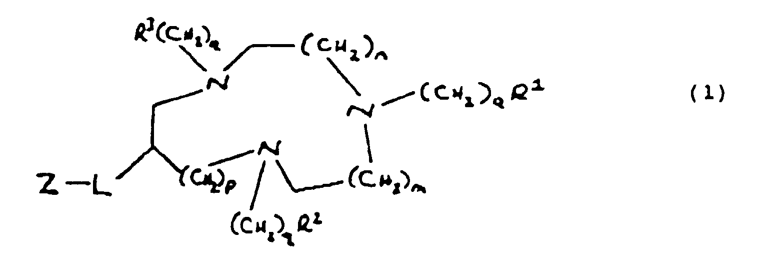 oder einen Metallkomplex davon mit einem Antikörper umfaßt.

14. Verfahren nach Anspruch 13, wobei R¹, R², R³, q und L wie in einem der Ansprüche 2, 3, 4 und 6 definiert sind.

15. Verfahren nach Anspruch 13 für die Herstellung einer Verbindung der Formel (2a): 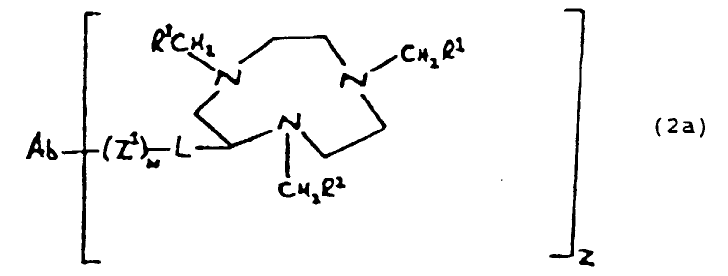 wobei
R¹, R² und R³, die gleich oder unterschiedlich sein können, jeweils ein Wasserstoffatom oder eine -CO₂H- oder -P(O)(XH)R⁴-Gruppe (worin X ein Sauerstoff- oder Schwefelatom ist und R⁴ ein Wasserstoffatom oder ein Alkyl- oder Alkoxyrest ist), sind, mit der Maßgabe, daß mindestens einer der Reste R¹, R² und R³ eine -P(O)(XH)R⁴-Gruppe ist,
L eine kovalente Bindung oder eine Linkergruppe ist,
Z¹ der Rest einer reaktiven funktionellen Gruppe, befähigt zur Reaktion mit einer Thiol-, Amino-, Carboxyl-, Hydroxyl-, Aldehyd-, aromatischen oder heteroaromatischen Gruppe, ist,
w Null oder eine Zahl 1 ist,
z eine Zahl 1 oder mehr ist,
Ab ein Antikörper ist,
und Metallkomplexe und/oder Salze davon.

16. Verfahren nach Anspruch 15, wobei R¹, R² und R³ jeweils eine -P(O)(OH)CH₃-Gruppe sind.

17. Verfahren nach Anspruch 15 oder 16, wobei L eine gegebenenfalls substituierte, aliphatische Kohlenwasserstoffkette, gegebenenfalls durch ein oder mehrere Heteroatome ausgewählt aus -O- oder -S-, oder durch eine oder mehrere -N(R⁵)-, -N(R⁵)CO-, cycloaliphatischen, aromatischen oder heteroaromatischen Gruppen unterbrochen, ist und Z¹ der Rest einer Gruppe, befähigt zur Reaktion mit einer Thiol-, Amino-, Carboxyl-, Aldehyd-, aromatischen oder heteroaromatischen Gruppe, ist, wobei die fakultativen Substituenten Halogenatome oder Gruppen, ausgewählt aus C₁₋₆Alkoxy, Hydroxy, Nitro, -N(R⁶)(R⁷) [worin R⁶ ein Wasserstoffatom oder ein C₁₋₆Alkylrest ist und R⁷ ein C₁₋₆Alkylrest ist], oder eine Gruppe der Formel -(CH₂)ₙCON(R⁸)(R⁹) [worin n Null oder eine Zahl von 1 bis einschließlich 4 ist, R⁸ ein Wasserstoffatom oder ein C₁₋₆Alkylrest ist und R⁹ ein C₁₋₆Alkylrest, gegebenenfalls substituiert durch ein oder mehrere Halogenatome, oder Nitro-, Amino- oder Hydroxygruppen ist] umfassen.

18. Verfahren nach einem der Ansprüche 13 bis 17, umfassend den Schritt der Komplexierung mit Indium oder Gallium.

## Revendications (Revendications pour l'(les) Etat(s) contractant(s) suivant(s): AT, BE, CH, LI, DE, DK, FR, GB, IT, LU, NL, SE)

1. Composé de formule (1) : 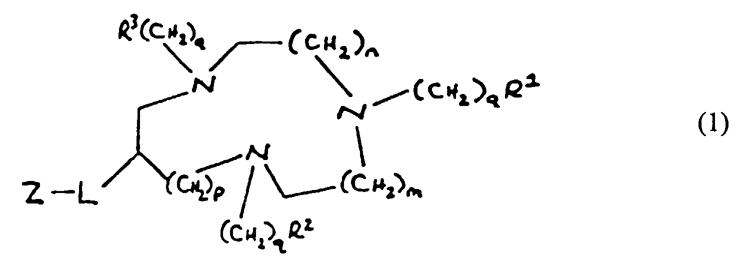 dans laquelle :
m et n, qui peuvent être identiques ou différents, valent chacun zéro ou représentent le nombre entier 1, 2 ou 3 ;
p vaut zéro ou représente le nombre entier 1 ou 2 ;
q vaut zéro ou représente un nombre entier compris entre 1 et 6, bornes comprises ;
R¹, R² et R³, qui peuvent être identiques ou différents, sont chacun un atome d'hydrogène ou un groupe -CO₂H ou -P(O)(XH)R⁴ (où X est un atome d'oxygène ou de soufre et R⁴ est un atome d'hydrogène ou un groupe alkyle ou alcoxy), du moment qu'au moins l'un de R¹, R² et R³ est un groupe -P(O)(XH)R⁴ ;
L est une liaison covalente ou un groupe de liaison ;
Z est un atome d'hydrogène ou un groupe fonctionnel réactif capable de réagir avec un groupe thiol, amino, carboxy, hydroxy, aldéhyde, aromatique ou hétéroaromatique ;
et ses sels et/ou complexes métalliques.

2. Composé selon la revendication 1, dans lequel R¹, R² et R³ sont identiques et sont chacun un groupe -P(O)(XH)R⁴.

3. Composé selon la revendication 2, dans lequel R¹, R² et R³ sont chacun un groupe -P(O)(OH)CH₃.

4. Composé selon l'une quelconque des revendications précédentes, dans lequel q représente le nombre entier 1.

5. Composé selon l'une quelconque des revendications précédentes, dans lequel L est une liaison covalente et Z est un atome d'hydrogène.

6. Composé selon l'une quelconque des revendications 1 à 4, dans lequel L est une chaîne hydrocarbonée aliphatique éventuellement substituée et éventuellement interrompue par un ou plusieurs hétéroatomes choisis parmi -O- et -S- ou par un ou plusieurs groupes -N(R⁵)-, -N(R⁵)CO-, cycloaliphatiques, aromatiques ou hétéroaromatiques,
dans lequel les substituants facultatifs comprennent des atomes d'halogène ou des groupes choisis parmi les groupes alcoxy en C₁ à C₆, hydroxy, nitro, -N(R⁶)(R⁷) [où R⁶ est un atome d'hydrogène ou un groupe alkyle en C₁ à C₆ et R⁷ est un groupe alkyle en C₁ à C₆], ou un groupe de formule -(CH₂)ₙCON(R⁸)(R⁹) [où n vaut zéro ou représente un nombre entier compris entre 1 et 4, bornes comprises, R⁸ est un atome d'hydrogène ou un groupe alkyle en C₁ à C₆ et R⁹ est un groupe alkyle en C₁ à C₆ éventuellement substitué par un ou plusieurs atomes d'halogène ou par un ou plusieurs groupes nitro, amino ou hydroxy].

7. Composé selon la revendication 1, de formule (1a) : 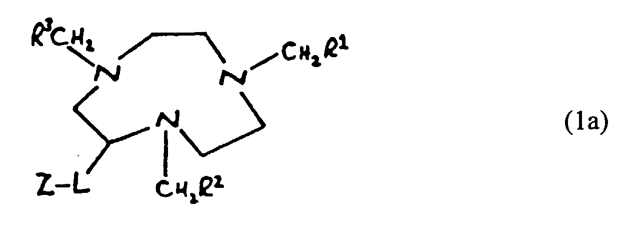 dans laquelle :
R¹, R² et R³, qui peuvent être identiques ou différents, sont chacun un atome d'hydrogène ou un groupe -CO₂H ou -P(O)(XH)R⁴ (où X est un atome d'oxygène ou de soufre et R⁴ est un atome d'hydrogène ou un groupe alkyle ou alcoxy), du moment qu'au moins l'un de R¹, R² et R³ est un groupe -P(O)(XH)R⁴ ;
L est une liaison covalente ou un groupe de liaison ;
Z est un atome d'hydrogène ou un groupe fonctionnel réactif capable de réagir avec un groupe thiol, amino, carboxy, hydroxy, aldéhyde, aromatique ou hétéroaromatique ;
et ses sels et/ou complexes métalliques.

8. Composé selon la revendication 7, dans lequel R¹, R² et R³ sont chacun un groupe -P(O)(OH)CH₃.

9. Composé selon la revendication 7 ou 8, dans lequel L est une chaîne hydrocarbonée aliphatique éventuellement substituée et éventuellement interrompue par un ou plusieurs hétéroatomes choisis parmi -O- et -S- ou par un ou plusieurs groupes -N(R⁵)-, -N(R⁵)CO-, cycloaliphatiques, aromatiques ou hétéroaromatiques, et Z est un groupe quelconque capable de réagir avec un groupe thiol, amino, carboxy, hydroxy, aldéhyde, aromatique ou hétéroaromatique,
dans lequel les substituants facultatifs comprennent des atomes d'halogène ou des groupes choisis parmi les groupes alcoxy en C₁ à C₆, hydroxy, nitro, -N(R⁶)(R⁷) [où R⁶ est un atome d'hydrogène ou un groupe alkyle en C₁ à C₆ et R⁷ est un groupe alkyle en C₁ à C₆], ou un groupe de formule -(CH₂)ₙCON(R⁸)(R⁹) [où n vaut zéro ou représente un nombre entier compris entre 1 et 4, bornes comprises, R⁸ est un atome d'hydrogène ou un groupe alkyle en C₁ à C₆ et R⁹ est un groupe alkyle en C₁ à C₆ éventuellement substitué par un ou plusieurs atomes d'halogène ou par un ou plusieurs groupes nitro, amino ou hydroxy].

10. Composé selon la revendication 7 ou 8, dans lequel L est une liaison covalente et Z est un atome d'hydrogène.

11. Complexe d'indium ou de gallium d'un composé selon l'une quelconque des revendications précédentes.

12. Complexe de gallium d'un composé selon la revendication 10.

13. Composé conjugué de formule (2) : 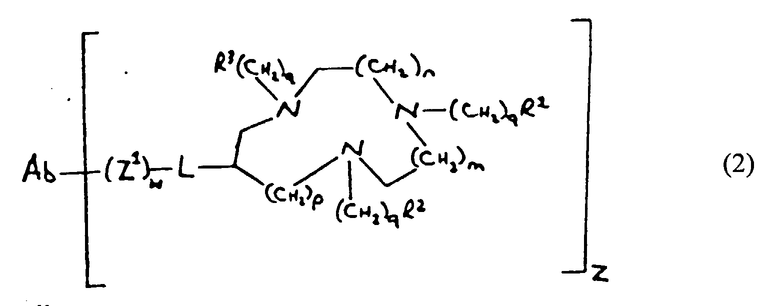 dans laquelle :
m et n, qui peuvent être identiques ou différents, valent chacun zéro ou représentent le nombre entier 1, 2 ou 3 ;
p vaut zéro ou représente le nombre entier 1 ou 2 ;
q vaut zéro ou représente un nombre entier compris entre 1 et 6, bornes comprises ;
R¹, R² et R³, qui peuvent être identiques ou différents, sont chacun un atome d'hydrogène ou un groupe -CO₂H ou -P(O)(XH)R⁴ (où X est un atome d'oxygène ou de soufre et R⁴ est un atome d'hydrogène ou un groupe alkyle ou alcoxy), du moment qu'au moins l'un de R¹, R² et R³ est un groupe -P(O)(XH)R⁴ ;
L est une liaison covalente ou un groupe de liaison ;
Z¹ est le résidu d'un groupe fonctionnel réactif capable de réagir avec un groupe thiol, amino, carboxy, hydroxy, aldéhyde, aromatique ou hétéroaromatique ;
w vaut zéro ou représente le nombre entier 1 ;
z est un nombre entier valant 1 ou plus ;
Ab est un anticorps ;
et ses sels et/ou complexes métalliques.

14. Composé conjugué selon la revendication 13, dans lequel R¹, R², R³, q et L sont tels que définis dans l'une quelconque des revendications 2, 3, 4 et 6.

15. Composé conjugué selon la revendication 13, de formule (2a) : 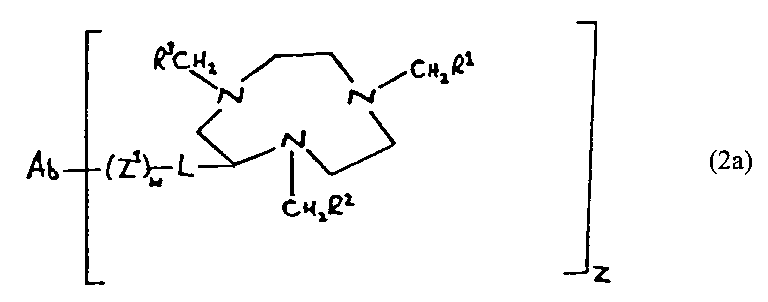 dans laquelle :
R¹, R² et R³, qui peuvent être identiques ou différents, sont chacun un atome d'hydrogène ou un groupe -CO₂H ou -P(O)(XH)R⁴ (où X est un atome d'oxygène ou de soufre et R⁴ est un atome d'hydrogène ou un groupe alkyle ou alcoxy), du moment qu'au moins l'un de R¹, R² et R³ est un groupe -P(O)(XH)R⁴ ;
L est une liaison covalente ou un groupe de liaison ;
Z¹ est le résidu d'un groupe fonctionnel réactif capable de réagir avec un groupe thiol, amino, carboxy, hydroxy, aldéhyde, aromatique ou hétéroaromatique ;
w vaut zéro ou représente le nombre entier 1 ;
z est un nombre entier valant 1 ou plus ;
Ab est un anticorps ;
et ses sels et/ou complexes métalliques.

16. Composé conjugué selon la revendication 15, dans lequel R¹, R² et R³ sont chacun un groupe -P(O)(OH)CH₃.

17. Composé conjugué selon la revendication 15 ou 16, dans lequel L est une chaîne hydrocarbonée aliphatique éventuellement substituée et éventuellement interrompue par un ou plusieurs hétéroatomes choisis parmi -O- et -S- ou par un ou plusieurs groupes -N(R⁵)-, -N(R⁵)CO-, cycloaliphatiques, aromatiques ou hétéroaromatiques, et Z¹ est le résidu d'un groupe quelconque capable de réagir avec un groupe thiol, amino, carboxy, hydroxy, aldéhyde, aromatique ou hétéroaromatique, dans lequel les substituants facultatifs comprennent des atomes d'halogène ou des groupes choisis parmi les groupes alcoxy en C₁ à C₆, hydroxy, nitro, -N(R⁶)(R⁷) [où R⁶ est un atome d'hydrogène ou un groupe alkyle en C₁ à C₆ et R⁷ est un groupe alkyle en C₁ à C₆], ou un groupe de formule -(CH₂)ₙCON(R⁸)(R⁹) [où n vaut zéro ou représente un nombre entier compris entre 1 et 4, bornes comprises, R⁸ est un atome d'hydrogène ou un groupe alkyle en C₁ à C₆ et R⁹ est un groupe alkyle en C₁ à C₆ éventuellement substitué par un ou plusieurs atomes d'halogène ou par un ou plusieurs groupes nitro, amino ou hydroxy].

18. Complexe d'indium ou de gallium d'un composé conjugué selon l'une quelconque des revendications 13 à 17.

## Revendications (Revendications pour l'(les) Etat(s) contractant(s) suivant(s): ES, GR)

1. Procédé pour la préparation d'un composé de formule (1) : 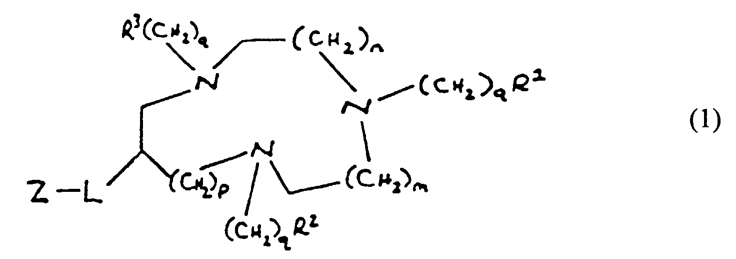 dans laquelle :
m et n, qui peuvent être identiques ou différents, valent chacun zéro ou représentent le nombre entier 1, 2 ou 3 ;
p vaut zéro ou représente le nombre entier 1 ou 2 ;
q vaut zéro ou représente un nombre entier compris entre 1 et 6, bornes comprises ;
R¹, R² et R³, qui peuvent être identiques ou différents, sont chacun un atome d'hydrogène ou un groupe -CO₂H ou -P(O)(XH)R⁴ (où X est un atome d'oxygène ou de soufre et R⁴ est un atome d'hydrogène ou un groupe alkyle ou alcoxy), du moment qu'au moins l'un de R¹, R² et R³ est un groupe -P(O)(XH)R⁴ ;
L est une liaison covalente ou un groupe de liaison ;
Z est un atome d'hydrogène ou un groupe fonctionnel réactif capable de réagir avec un groupe thiol, amino, carboxy, hydroxy, aldéhyde, aromatique ou hétéroaromatique ;
et de ses sels et/ou complexes métalliques ;
le procédé comprenant l'étape consistant à faire réagir un composé de formule (3) : 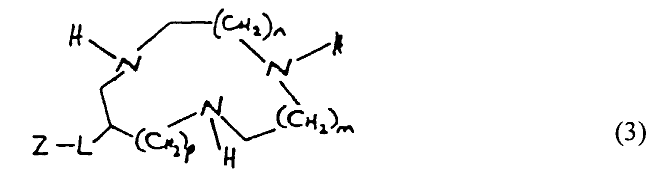 ou un complexe métallique de celui-ci, avec un réactif D(CH₂)_{q}P(O)(OH)R⁴, D(CH₂)_{q}H ou D(CH₂)_{q}CO₂H (ou des formes de celui-ci acide-protégées), où D est un groupe déplaçable.

2. Procédé selon la revendication 1, dans lequel R¹, R² et R³ sont identiques et sont chacun un groupe -P(O)(XH)R⁴.

3. Procédé selon la revendication 2, dans lequel R¹, R² et R³ sont chacun un groupe -P(O)(OH)CH₃.

4. Procédé selon l'une quelconque des revendications précédentes, dans lequel q représente le nombre entier 1.

5. Procédé selon l'une quelconque des revendications précédentes, dans lequel L est une liaison covalente et Z est un atome d'hydrogène.

6. Procédé selon l'une quelconque des revendications 1 à 4, dans lequel L est une chaîne hydrocarbonée aliphatique éventuellement substituée et éventuellement interrompue par un ou plusieurs hétéroatomes choisis parmi -O- et -S- ou par un ou plusieurs groupes -N(R⁵)-, -N(R⁵)CO-, cycloaliphatiques, aromatiques ou hétéroaromatiques,
dans lequel les substituants facultatifs comprennent des atomes d'halogène ou des groupes choisis parmi les groupes alcoxy en C₁ à C₆, hydroxy, nitro, -N(R⁶)(R⁷) [où R⁶ est un atome d'hydrogène ou un groupe alkyle en C₁ à C₆ et R⁷ est un groupe alkyle en C₁ à C₆], ou un groupe de formule -(CH₂)ₙCON(R⁸)(R⁹) [où n vaut zéro ou représente un nombre entier compris entre 1 et 4, bornes comprises, R⁸ est un atome d'hydrogène ou un groupe alkyle en C₁ à C₆ et R⁹ est un groupe alkyle en C₁ à C₆ éventuellement substitué par un ou plusieurs atomes d'halogène ou par un ou plusieurs groupes nitro, amino ou hydroxy].

7. Procédé selon la revendication 1, de formule (1a) : 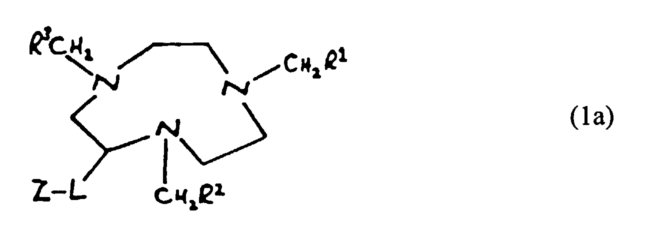 dans laquelle :
R¹, R² et R³, qui peuvent être identiques ou différents, sont chacun un atome d'hydrogène ou un groupe -CO₂H ou -P(O)(XH)R⁴ (où X est un atome d'oxygène ou de soufre et R4 est un atome d'hydrogène ou un groupe alkyle ou alcoxy), du moment qu'au moins l'un de R¹, R² et R³ est un groupe -P(O)(XH)R⁴ ;
L est une liaison covalente ou un groupe de liaison ;
Z est un atome d'hydrogène ou un groupe fonctionnel réactif capable de réagir avec un groupe thiol, amino, carboxy, hydroxy, aldéhyde, aromatique ou hétéroaromatique ;
et ses sels et/ou complexes métalliques.

8. Procédé selon la revendication 7, dans lequel R¹, R² et R³ sont chacun un groupe -P(O)(OH)CH₃.

9. Procédé selon la revendication 7 ou 8, dans lequel L est une chaîne hydrocarbonée aliphatique éventuellement substituée et éventuellement interrompue par un ou plusieurs hétéroatomes choisis parmi -O- et -S- ou par un ou plusieurs groupes -N(R⁵)-, -N(R⁵)CO-, cycloaliphatiques, aromatiques ou hétéroaromatiques, et Z est un groupe quelconque capable de réagir avec un groupe thiol, amino, carboxy, hydroxy, aldéhyde, aromatique ou hétéroaromatique,
dans lequel les substituants facultatifs comprennent des atomes d'halogène ou des groupes choisis parmi les groupes alcoxy en C₁ à C₆, hydroxy, nitro, -N(R⁶)(R⁷) [où R⁶ est un atome d'hydrogène ou un groupe alkyle en C₁ à C₆ et R⁷ est un groupe alkyle en C₁ à C₆], ou un groupe de formule -(CH₂)ₙCON(R⁸)(R⁹) [où n vaut zéro ou représente un nombre entier compris entre 1 et 4, bornes comprises, R⁸ est un atome d'hydrogène ou un groupe alkyle en C₁ à C₆ et R⁹ est un groupe alkyle en C₁ à C₆ éventuellement substitué par un ou plusieurs atomes d'halogène ou par un ou plusieurs groupes nitro, amino ou hydroxy].

10. Procédé selon la revendication 7 ou 8, dans lequel L est une liaison covalente et Z est un atome d'hydrogène.

11. Procédé selon l'une quelconque des revendications précédentes, comprenant l'étape de complexation avec de l'indium ou du gallium.

12. Procédé selon la revendication 11, comprenant l'étape de complexation avec du gallium.

13. Procédé pour la préparation d'un composé conjugué de formule (2) : 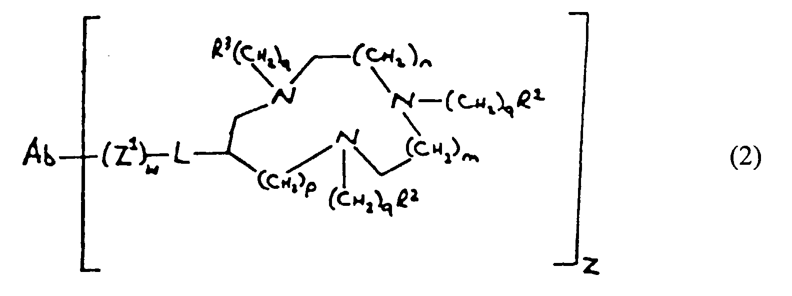 dans laquelle :
m et n, qui peuvent être identiques ou différents, valent chacun zéro ou représentent le nombre entier 1, 2 ou 3 ;
p vaut zéro ou représente le nombre entier 1 ou 2 ;
q vaut zéro ou représente un nombre entier compris entre 1 et 6, bornes comprises ;
R¹, R² et R³, qui peuvent être identiques ou différents, sont chacun un atome d'hydrogène ou un groupe -CO₂H ou -P(O)(XH)R⁴ (où X est un atome d'oxygène ou de soufre et R⁴ est un atome d'hydrogène ou un groupe alkyle ou alcoxy), du moment qu'au moins l'un de R¹, R² et R³ est un groupe -P(O)(XH)R⁴ ;
L est une liaison covalente ou un groupe de liaison ;
Z¹ est le résidu d'un groupe fonctionnel réactif capable de réagir avec un groupe thiol, amino, carboxy, hydroxy, aldéhyde, aromatique ou hétéroaromatique ;
w vaut zéro ou représente le nombre entier 1 ;
z est un nombre entier valant 1 ou plus ;
Ab est un anticorps ;
et de ses sels et/ou complexes métalliques ;
le procédé comprenant l'étape consistant à faire réagir un composé de formule (1) : 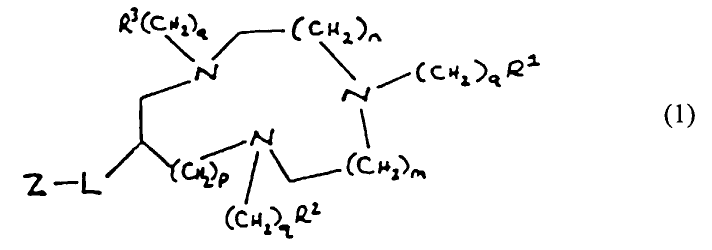 ou un complexe métallique de celui-ci, avec un anticorps.

14. Procédé selon la revendication 13, dans lequel R¹, R², R³, q et L sont tels que définis dans l'une quelconque des revendications 2, 3, 4 et 6.

15. Procédé selon la revendication 13, pour la préparation d'un composé de formule (2a) : 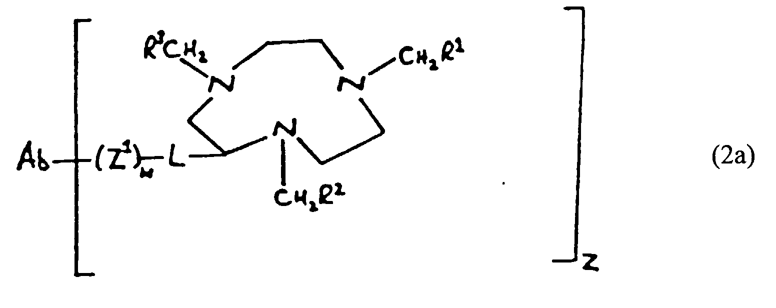 dans laquelle :
R¹, R² et R³, qui peuvent être identiques ou différents, sont chacun un atome d'hydrogène ou un groupe -CO₂H ou -P(O)(XH)R⁴ (où X est un atome d'oxygène ou de soufre et R⁴ est un atome d'hydrogène ou un groupe alkyle ou alcoxy), du moment qu'au moins l'un de R¹, R² et R³ est un groupe -P(O)(XH)R⁴ ;
L est une liaison covalente ou un groupe de liaison ;
Z¹ est le résidu d'un groupe fonctionnel réactif capable de réagir avec un groupe thiol, amino, carboxy, hydroxy, aldéhyde, aromatique ou hétéroaromatique ;
w vaut zéro ou représente le nombre entier 1 ;
z est un nombre entier valant 1 ou plus ;
Ab est un anticorps ;
et de ses sels et/ou complexes métalliques.

16. Procédé selon la revendication 15, dans lequel R¹, R² et R³ sont chacun un groupe -P(O)(OH)CH₃.

17. Procédé selon la revendication 15 ou 16, dans lequel L est une chaîne hydrocarbonée aliphatique éventuellement substituée et éventuellement interrompue par un ou plusieurs hétéroatomes choisis parmi -O- et -S- ou par un ou plusieurs groupes -N(R⁵)-, -N(R⁵)CO-, cycloaliphatiques, aromatiques ou hétéroaromatiques, et Z¹ est le résidu d'un groupe quelconque capable de réagir avec un groupe thiol, amino, carboxy, hydroxy, aldéhyde, aromatique ou hétéroaromatique, dans lequel les substituants facultatifs comprennent des atomes d'halogène ou des groupes choisis parmi les groupes alcoxy en C₁ à C₆, hydroxy, nitro, -N(R⁶)(R⁷) [où R⁶ est un atome d'hydrogène ou un groupe alkyle en C₁ à C₆ et R⁷ est un groupe alkyle en C₁ à C₆], ou un groupe de formule -(CH₂)ₙCON(R⁸)(R⁹) [où n vaut zéro ou représente un nombre entier compris entre 1 et 4, bornes comprises, R⁸ est un atome d'hydrogène ou un groupe alkyle en C₁ à C₆ et R⁹ est un groupe alkyle en C₁ à C₆ éventuellement substitué par un ou plusieurs atomes d'halogène ou par un ou plusieurs groupes nitro, amino ou hydroxy].

18. Procédé selon l'une quelconque des revendications 13 à 17, comprenant l'étape de complexation avec de l'indium ou du gallium.
